(19) Europäisches Patentamt
European Patent Office
Office européen des brevets

(11) Publication number: **0 059 645**
**B1**

(12) # EUROPEAN PATENT SPECIFICATION

(45) Date of publication of patent specification: **04.06.86**

(51) Int. Cl.$^4$: **C 12 Q 1/34,** C 12 Q 1/18

(21) Application number: **82301078.0**

(22) Date of filing: **03.03.82**

(54) **Method and kit for identification of beta-lactamases.**

(30) Priority: **03.03.81 GB 8106702**
**18.11.81 GB 8134734**

(43) Date of publication of application:
**08.09.82 Bulletin 82/36**

(45) Publication of the grant of the patent:
**04.06.86 Bulletin 86/23**

(84) Designated Contracting States:
**BE CH DE FR IT LI NL**

(56) References cited:
**GB-A-1 408 391**
**GB-A-2 026 689**

**CHEMICAL ABSTRACTS, vol. 85, no. 21, November 22, 1976, page 89, abstract 154496h, COLUMBUS OHIO (US), D.F. MAHONEY et al. "Substrate inhibition of beta-lactamases, a method for predicting euzymic stability of cephalosporins"**

(73) Proprietor: **NATIONAL RESEARCH DEVELOPMENT CORPORATION**
**101 Newington Causeway**
**London SE1 6BU (GB)**

(72) Inventor: **James, Richard**
**Compass Cottage Besthorpe**
**Attleborough Norfolk NR17 2NZ (GB)**

(74) Representative: **Percy, Richard Keith**
**Patent Department National Research Development Corporation 101 Newington Causeway**
**London SE1 6BU (GB)**

(56) References cited:
**CHEMICAL ABSTRACTS, vol. 94, no. 21, May 25, 1981, page 288, abstract 169966w, COLUMBUS OHIO (US), P. SCHINDLER et al. "Use of PADAC a novel chromogenic Beta-lactamase substrate, for the detection of beta-lactamase producing organisms and assay of beta-lactamase inhibitors/inactivators"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

(56) References cited:

**CHEMICAL ABSTRACTS, vol. 93, no. 1, July 7, 1980, page 285, abstract 2921v, COLUMBUS OHIO (US), R. GUAY et al. "Comparative efficiency of inhibitors on beta-lactamase produced by Gram-negative bacteria"**

**CHEMICAL ABSTRACTS, vol. 93, no. 13, September 29, 1980, page 286, abstract 127834a, COLUMBUS OHIO (US), S. YAMABE "Spectrophotometric determination of inhibitory effects of CP-45899 on beta-lactamase with benzylpenicillin and nitrocefin"**

**Description**

This invention relates to an assay method for identifying or assisting in identifying beta-lactamase enzymes, especially in bacterial extracts, and a kit for use in the assay method.

Many antibiotics, particularly of the penicillin and cephalosporin classes, contain a beta-lactam ring of formula:—

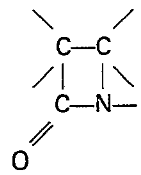

and are conveniently referred to as beta-lactam antibiotics. These antibiotics have been used for many years to treat bacterial infections. However, bacteria showing marked resistance to several beta-lactam antibiotics have evolved. This resistance is now widespread among many genera of bacteria. Meanwhile, new beta-lactam antibiotics are being developed.

The most common mechanism by which bacteria resist the beta-lactam antibiotics is the production of beta-lactamase enzymes (beta-lactamases). Beta-lactamases hydrolyse the beta-lactam group, causing ring opening and therapeutic de-activation of the antibiotic. Several distinct beta-lactamases, coded for by plasmids, have been identified in different gram-negative bacteria, see Matthew, Hedges and Smith in J. Bacteriol. *138*, 657—662 (1979). The identification was carried out on the basis of their rates of hydrolysis on a range of beta-lactam substrates, together with isoelectric point and molecular weight determination. The relative rates of hydrolysis were monitored by titration. Differences in the rates were found often (but not always) to be characteristic of different beta-lactamases. Equally, the different beta-lactam substrates have different rates of hydrolysis for the same beta-lactamase. The determination of hydrolysis rates is very laborious, particularly since multiple titrations are required. Such a method is time-consuming and open to human error. It is also insensitive in cases in which the beta-lactam substrate has a relatively high affinity for the enzyme under test. Another problem is that in many cases it is impossible to distinguish whether a low rate of hydrolysis should be ascribed to low affinity of the enzyme for the beta-lactam substrate or to inhibition of the enzyme by the substrate.

Isoelectric point determination of beta-lactamases has been carried out by isoelectric focussing. See, for example, Matthew *et al* in Journal of General Microbiology (1975), *88*, 169. In this method a sheet of polyacrylamide gel containing ampholytes is connected to electrodes. The sample of unknown beta-lactamase is applied to the gel near the anode. A pH gradient is produced in the gel electrophoretically and the enzyme migrates to that part of the gel having a pH corresponding to the isoelectric point (pI) mf the enzyme. The enzyme "focusses" into sharply defined bands at the pI area. The focussing force is more powerful than any tendency to diffuse. Isoelectric focussing has various disadvantages. It is difficult to automate, large numbers of samples are required and it is expensive and time-consuming. Since different enzymes often have similar pIs, it is not always easy to distinguish between enzymes on the basis of isoelectric focussing alone. It has therefore often been found necessary to use both isoelectric focussing and determination of hydrolysis rates of beta-lactam substrates in order to identify beta-lactamases.

Existing kits for identifying beta-lactamases are effective only for a very few of them.

In the field of beta-lactam therapy, it has long been a problem that no one single beta-lactam is effective to combat all the clinically important beta-lactamases, see, for example, the last paragraph of the paper by C. H. O'Callaghan and A. Morris, Antimicrobial Agents and Chemotherapy *2*, 442—448 (1972).

A partial solution to the problem arose with the discovery of new beta-lactams having an inhibitory effect on penicillinases and cephalosporinases. Many studies have been made of the relative effectiveness of beta-lactam antibiotics as inhibitors of beta-lactamases. In one recent such study, R. Guay *et al.*, IRCS Medical Science *8*, 209 (1980), the relative inhibiting effectiveness of 4 beta-lactam antibiotics on 13 beta-lactamases was compared by a competitive assay of the antibiotic with nitrocefin. The results are presented in terms of a ratio of Ki/Km, where Ki is the inhibition constant and Km the Michaelis constant. The observed wide range of the Ki/Km values demonstrated that the inhibition activity of the 4 antibiotics depended on the particular beta-lactamase. The two cephalosporin antibiotics were found to be more potent than the two penicillin antibiotics tested.

It has now been found that beta-lactamases can be identified by an assay which is rapid and simple to perform.

The invention is concerned with a competitive assay in which a reference beta-lactam-containing substrate having or acquiring as a result of the assay a distinctive spectral characteristic in the visible light region of the spectrum (a "chromogenic substrate"), and another substrate (a "competing substrate"), usually another beta-lactam, compete for a limited quantity of a beta-lactamase. The assay depends upon competition between two reactions. The first reaction is between the chromogenic substrate, for example nitrocefin, and the beta-lactamase enzyme. Nitrocefin is a cephalosporin which has a yellow colour. Hydrolysis by a beta-lactamase caused opening of the lactam ring, producing a bathochromic shift in its

absorption spectrum and consequently a change of colour to red. The second, competing reaction between the beta-lactamase and the competing substrate, for example a beta-lactam, will depending upon its extent, inhibit formation of this red colour. For any specific concentration of competing beta-lactam, the degree of bathochromic shift which has taken place will be a function of the relative affinity of the beta-lactamase for that substrate.

The invention includes specifically a method of identifying an unidentified beta-lactamase, which method comprises performing a competitive assay on said beta-lactamase with (1) a chromogenic beta-lactamase, monitoring the assay for a change, resulting from the assay, in the visible light spectrum of the chromogenic substrate, determining from this change a relative affinity of the competing substrate to the chromogenic substrate for the unidentified beta-lactamase, repeating said assay at least four times using for each assay a different competing substrate or the same competing substrate in no more than two different ratios of the competing substrate to the chromogenic substrate, and identifying the beta-lactamase by reference to a compilation of previously determined, relative affinities of each competing substrate relative to the chromogenic substrate for different beta-lactamases.

The invention also includes kits for use in the method, the kits being defined hereinafter.

The use of nitrocefin in the detection of beta-lactamases generically, i.e. without identifying the kind of beta-lactamase, is not new, see British Patent Specification No. 1,408,391 and numerous literature papers from 1972 onwards. Also, competitive assays using nitrocefin with other beta-lactams against known beta-lactamases have been used to compare the relative effectiveness of beta-lactams in therapy. Recently another chromogenic substrate for beta-lactamases, known as "PADAC" has been described and suggested for the detection of beta-lactamases generically see P. Schindler and G. Huber, Enzyme Inhibitors, Proc. Meet., edited by Brodbeck and Urs, Verlag Chemie 1980, pages 169—176, abstracted at Chemical Abstracts *94*, 169966w. What is novel and inventive comprises in combination the finding that the series of competitive assays on known beta-lactamases using a chromogenic substrate, e.g. nitrocefin, and a large number of competing substrates for the beta-lactamase, normally a beta-lactam, gives results which form a data bank of unexpected value; and the use of this data bank to identify beta-lactamases (rather than to compare the efficacy of beta-lactams). Such a data bank allows the man skilled in the art to determine that by using at least five competitive assays with different substrates (or the same substrate at different ratios to the chromogenic substrate) it will be possible to identify individual beta-lactamases.

The chromogenic substrate can be any which is capable of undergoing a change in its visible light spectrum when reacted under the competitive assay conditions with beta-lactamases, i.e. with at least those beta-lactamases from which the unidentified beta-lactamase is required to be identified. The chromogenic substrate has a visible colour *per se* or upon hydrolysis or, most conveniently, both. It is usually convenient to monitor a new absorption resulting from the reaction, but it is also possible to monitor the change in its original absorption. A shift in absorption caused by the reaction can be auxochromic or bathochromic.

The preferred chromogenic substrate for use in the present invention is nitrocefin. Nitrocefin is the E-isomer of 3 - (2,4 - dinitrostyryl) - (6*R*,7*R*) - 7 - (2 - thienylacetamido)ceph - 3 - em - 4 - carboxylic acid. It has an absorption maximum at approximately 386 nm. which is responsible for its yellow colour. On hydrolysis of its beta-lactam ring there is a bathochromic shift, giving an absorption maximum of wavelength some 100 nm longer, i.e. about 486 nm. This shift is responsible for the red colour. The relationship (explained hereinafter) between enzyme concentration and optical density at about 486 nm is linear over quite a wide range of initial concentration of enzyme, typically 0.006 to 0.2 enzyme units. It is therefore preferred to monitor the assay spectroscopically, determining the absorption at a wavelength in the region of 486 nm.

Although nitrocefin is greatly preferred, other chromogenic beta-lactam-containing substrates could be used. For example, N-(2-furyl)acryloyl penicillin shows a fall in absorbance at 330 nm. on hydrolysis, Durkin *et al*, J. Antibiot., *30*, 883—885 (1977). Another chromogenic substrate is described by Knusel *et al.* in Antimicrobial Agents and Chemotherapy, 140—149 (1976) and could be used in the assay of the invention. Of course, not every chromogenic substrate is necessarily useful with every beta-lactamase, but nitrocefin has displayed the desired high affinity for, and reversible attachment to, a wide variety of beta-lactamases. The new chromogenic cephalosporin "PADAC" (Hoechst AG), described as a 3 - position - pyridinium - 2 - azo - *p* - dimethylaniline - substituted cephalosporin, can also be used in the present invention, but it has a lower affinity for beta-lactamases than nitrocefin and is apparently not cleaved by one of the common clinical beta-lactamases, OXA-1. Example 10 illustrates its use in more detail.

The competing substrate can be any compound which competes with the chromogenic substrate for the beta-lactamase and, self-evidently, which does not mask the spectral change of the chromogenic substrate. In practice it will normally be a non-chromogenic beta-lactam, e.g. a penicillin, cephalosporin or thienamycin.

The invention includes two main embodiments. In the first embodiment the assays are monitored to give a quantitative relative affinity. This is conveniently done by a spectrophotometer which gives in the first instance a series of readings of optical densities. Each "assay" consists of a series of individual experiments carried out using different concentrations of competing substrate. From the readings there is calculated, by computer, a single numerical relative affinity which applies to the particular combination of

beta-lactamase, chromogenic substrate and competing substrate and at a particular chosen temperature of assay. These numerical relative affinities have widely separated values differing according to the beta-lactamase and the beta-lactam. In the second embodiment the relative affinity is determined semi-quantitatively and the assay is monitored by visual inspection. Typically, each assay consists of only one experiment, using a carefully predetermined concentration of competing substrate. The concentration is selected so as to give a visually recognisable difference in colour as between different beta-lactamases and different beta-lactams. This method therefore produces a semi-quantitative relative affinity which applies to the particular combination of beta-lactamase, chromogenic substrate, competing substrate and concentration of competing substrate used. This second embodiment comprises a simple visual method monitored merely by eye or a sophisticated visual method monitored by spectrophotometer.

Referring first to the first embodiment, i.e. the "quantitative method", a preferred way to compile relative affinities and to carry out the assay is to make a seris of determinations of change in optical density corresponding to competition by different concentrations of competing substrate and from these results determine a relative affinity after an arbitrarily chosen time of reaction. It is convenient to express the results in terms of a Relative Substrate Affinity Index (RSAI) value.

$$\text{RSAI} = \frac{\text{Concentration of competing substrate which gives 50\% inhibition of ring-opening hydrolysis of the chromogenic substrate}}{\text{Concentration of chromogenic substrate in the assay}}$$

A high RSAI value therefore indicates a low affinity of the beta-lactamase for that competing substrate, and therefore predicts resistance of a beta-lactam competing substrate to hydrolysis by the beta-lactamase. Low RSAI values, in contrast, predict sensitivity to hydrolysis by a beta-lactamase.

Of course, the relative affinity could be expressed in any other appropriate way. In particular any other degree of inhibition could be substituted for the 50% degree chosen above, subject of course to the demands of accuracy in measurement and distinguishing adequately between different competing substrates whenever possible.

Since different beta-lactam substrates compete with the chromogenic substrate for a given beta-lactamase at widely different concentrations, it might be possible to identify a beta-lactamase on the basis of the invention using a single competing beta-lactam. If necessary, several different competing beta-lactams can be used. Each such beta-lactam will usually have a different "profile" determined by its relative affinity towards a plurality of different beta-lactamases.

In one aspect of the invention advantage is taken of the probability that the beta-lactamase to be identified will have a profile which is unique for a small number, preferably 1 to 5 competing substrates. To understand the method more fully the reader is referred to Table 1 below in which a compilation of RSAI values is tabulated for nitrocefin as chromogenic substrate. To compile Table 1, assays were performed as described in Example 1 for 26 different competing beta-lactam substrates and 17 different beta-lactamase enzymes. All these beta-lactam substrates are known compounds and their systematic names are given in the Index Guide for Chemical Abstracts published by the American Chemical Society. The newer ones will be found in the 1977—80 Cumulative Index Guide Supplement. Table 1 also gives some isoelectric points and Michaelis constants.

Of the 17 beta-lactamases in Table 1, only 11 are currently produced on a significant scale by ordinary bacteria. These are TEM-1, TEM-2, SHV-1, HMS-1, OXA-1 to 3 and PSE-1 to 4 (numbered 1—7 and 10—13 in the Table). Of the three chromosomally-coded enzymes *Enterobacter cloacae* (8) is not very important clinically, *E. coli* SNU 35 (9) is an artificially produced organism and *Pseudomonas aeruginosa* 1937E (14) is induced by ampicillin treatment. *Klebsiella* normally produce SHV-1, TEM-1 or a mixture of the two. The *Klebsiella*-derived enzymes listed in the Table have been obtained from clinical isolates but they are rare.

K 10317 is a *Klebsiella aerogenes* from the UK National Collection of Type Cultures.

K 1073 is a *Klebsiella* of capsular serotype 21 obtained from a patient in the West Midlands in 1980 and characterised by the Coventry Public Health Laboratory. K17 is a *Klebsiella* of capsular serotype 17 obtained from New Zealand in 1980 and kindly supplied by Professor Mary Cooke, Microbiology Department, Leeds University.

TABLE 1: RSAI values (nitrocefin)

| Enzyme No. | Bacterial origin of the enzyme | Beta-lactamase enzyme | Isoelectric point of enzyme | $K_m$ μg/ml (nitrocefin) |
|---|---|---|---|---|
| 1. | *E. coli* 2136E | TEM-1 | 5.4 | — |
| 2. | *E. coli* 1725E | TEM-2 | 5.6 | 25 |
| 3. | *E. coli* 2008E | SHV-1 | 7.6 | 16 |
| 4. | *E. coli* 1946 | HMS-1 | 5.2 | — |
| 5. | *E. coli* 1527E | OXA-1 | 7.4 | 9 |
| 6. | *E. coli* R46 | OXA-2 | 7.6ϕ | <5 |
| 7. | *E. coli* R57B | OXA-3 | 7.1 | 28 |
| 8. | *E. cloacae* | Chromosomal, constitutive | — | — |
| 9. | *E. coli* SNU35 | Chromosomal, hyper-induced | — | 18 |
| 10. | *Ps. aerug.* 1937E | PSE-1 | 5.7 | — |
| 11. | *Ps. aerug.* 1973E | PSE-2 | — | — |
| 12. | *Ps. aerug.* 1920E | PSE-3 | — | — |
| 13. | *Ps. aerug.* 1559E | PSE-4 | — | — |
| 14. | *Ps. aerug.* 1937E+ ampicillin | Chromosomal, induced | — | — |
| 15. | *K. aerog.* 10317 | | — | — |
| 16. | *Klebsiella* 1073 | | — | — |
| 17. | *Klebsiella* 17 | | — | — |

ϕ mean of two determinations

TABLE 1: RSAI values (nitrocefin) continued

| Enzyme No. | Penicillin G | Ampicillin | Cloxacillin | Cephalothin | Cephalozolin | Cephaloridine | Cefoxitin |
|---|---|---|---|---|---|---|---|
| 1. | 3 | 4 | 0.75 | 10 | 30 | 40 | 60* |
| 2. | 1 | 2.6 | 0.5 | 15 | 46 | 20 | 80* |
| 3. | 1 | 3 | 2 | 7 | 15 | 30 | 600 |
| 4. | 0.5 | 0.5 | 0.02 | 1 | 0.8 | 6 | 60 |
| 5. | 0.2 | 4 | 0.6 | 4 | 10 | 60 | 20 |
| 6. | 0.6 | 10 | 30 | 0.7 | 3.4 | 4 | 0.2 |
| 7. | 0.4 | 0.2 | 0.01 | 0.4 | 3.4 | 4.4 | 0.004 |
| 8. | 0.08 | 0.02 | 0.00004 | 0.6 | 17 | 16 | 0.01 |
| 9. | 0.18 | 0.03* | 0.0000016* | 0.8 | 46 | 2* | 0.0008 |
| 10. | 0.6 | 0.4 | 2 | 4 | 2 | 6 | 20 |
| 11. | 0.2 | 0.85 | 4.5 | 0.54 | 1.4 | 22 | 24 |
| 12. | 0.3 | 1.7 | 0.24 | 0.76 | 3.2 | 2.2 | 11.4 |
| 13. | 0.48 | 2.7 | 3.3 | 6 | 2.6 | 3.5 | 27 |
| 14. | 0.13 | 0.12 | 0.00017 | 0.7 | 15 | 1.5ϕ | 0.003 |
| 15. | 2.2 | 9.4 | 3.7* | 7 | 30 | 30 | 350* |
| 16. | 2 | 6.7 | 4* | 5.8 | 16 | 19.2 | 1020 |
| 17. | 1.2 | 2.9 | 14 | 1.8 | 1 | 5.4 | 1.6 |

ϕ mean of two determinations
* shows irreversible inhibition

TABLE 1: RSAI values (nitrocefin) continued

| Enzyme | Cefuroxime | Cefotaxime | SQ. 14,359 | Moxalactam | Cefopera-zone | Carbeni-cillin |
|---|---|---|---|---|---|---|
| 1. | 50 | 400 | 10* | 40* | 8 | 0.6 |
| 2. | 40 | 600 | 15* | 60* | 10 | 0.4 |
| 3. | 20 | 400 | 45* | 280 | 4.5 | 0.5 |
| 4. | 16 | 11 | 1.5* | 0.3* | 0.2 | 0.09 |
| 5. | 10 | 9 | 50* | 4* | 4.5 | 0.5 |
| 6. | 3.6 | 12 | 0.7* | 3* | 2 | 15 |
| 7. | 0.003 | 15 | 0.6* | 1* | 2 | 12 |
| 8. | 0.0002 | 0.0006 | 0.002 | 0.0001* | 1.3 | 0.0003* |
| 9. | 0.002 | 0.004 | 0.003 | 0.002* | 1.9 | 0.00005* |
| 10. | 50 | 70 | 1.8 | 3.5 | 0.019 | 1.4 |
| 11. | 1.7 | 9.6 | 4.5 | 8.2 | 0.48 | 5.5 |
| 12. | 12 | 100 | 0.1 | 7 | 0.07 | 1.35 |
| 13. | 34 | 50 | 0.94 | 4.3 | 0.014 | 3 |
| 14. | 0.0001 | 0.004 | 0.004 | 0.006* | 0.5 | 0.008 |
| 15. | 14 | 320 | 29 | 60* | 5 | 0.7 |
| 16. | 12 | 330 | 60 | >80 | 2.2 | 0.5 |
| 17. | 2.3 | 60 | 7 | 3.5 | 0.006 | 0.4 |

* shows irreversible inhibition

TABLE 1: RSAI values (nitrocefin) continued

| Enzyme No. | Cefamandole | Cephradine | Mecillinam | Ceftizoxime | Ceftazidime (GR 20,263) | R0-13 9904+ | CP 45,899 |
|---|---|---|---|---|---|---|---|
| 1. | 11 | 35 | 90 | 1000 | >400 | 400 | 0.02 |
| 2. | 11 | 50 | 80 | 1000 | 1500 | 600 | 0.04* |
| 3. | 6.6 | 15 | 80 | 1400 | 1140 | 160 | 0.018* |
| 4. | 0.5 | 25 | 2 | 66 | 10 | 14 | 0.025* |
| 5. | 1.5 | 420 | 400 | 50 | >2000 | 0.9 | 8* |
| 6. | 1.7 | 86 | 100 | 110 | 900 | 10 | 0.15* |
| 7. | 0.75 | 100 | 30 | 200 | >400 | 3 | 0.05* |
| 8. | 0.03 | 1.8 | 1.3* | 0.1 | 0.1* | 0.0003* | 6.5* |
| 9. | 0.12* | 0.12 | 0.7* | 1.7 | 0.2* | 0.01 | 1.7* |
| 10. | 6.9 | 46 | 66 | 360 | 600 | 14 | 0.036 |
| 11. | 0.06 | 4.2 | 450 | 128 | >400 | 0.76 | 16 |
| 12. | 0.006 | 3.6 | 15 | 120 | 144 | 36 | 0.0014 |
| 13. | 9 | 80 | 140 | 380 | 420 | 42 | 0.015* |
| 14. | 5 | 1 | 68 | 500 | 0.46* | 38 | 2.5* |
| 15. | 5 | 18 | 80 | 630 | N 1000 | 74 | 0.08 |
| 16. | 3.9 | 25 | 102 | 720 | 1420 | 116 | 0.18 |
| 17. | 1.8 | 10.4 | 260 | 220 | 880 | 4 | 0.5 |

* shows irreversible inhibition
+ "R0-13-9904-001" was actually used.

TABLE 1: RSAI values (nitrocefin) continued

| Enzyme No. | Clavulanic acid | MK 0787 (N-Formimidoyl-thienamycin) | Amoxy-cillin | Ticar-cillin | Azlo-cillin | Mezlo-cillin |
|---|---|---|---|---|---|---|
| 1. | 0.003* | 0.1 | 1.3 | 0.035 | 1 | 3.6 |
| 2. | 0.002* | 0.1 | 2.2 | 0.49 | 1.1 | 6.3 |
| 3. | 0.006* | 1.2 | 2.8 | 0.23 | 1.6 | 3 |
| 4. | 0.006* | 1.2 | 0.8 | 0.175 | 0.42 | 0.9 |
| 5. | 0.02* | 0.6* | 1.2 | 0.65 | 1.4 | 1.9 |
| 6. | 0.9* | 0.005* | 8 | 26 | 19 | 19.4 |
| 7. | 1.6* | 0.0016* | 8.6 | 12 | 9 | 19.2 |
| 8. | 50 | 0.03 | 0.05 | 0.0008* | 0.05 | 0.4 |
| 9. | 4 | 2 | 0.02 | 0.001 | 0.02 | 0.3 |
| 10. | 0.0001 | 19.4 | 0.9 | 0.63 | 0.23 | 1.2 |
| 11. | 0.4 | 0.08 | 2.2 | 2.4 | 2.64 | 4.75 |
| 12. | 0.014 | 0.5 | 1.5 | 2 | 0.54 | 0.8 |
| 13. | 0.004* | 15 | 1 | 0.65 | 0.36 | 0.9 |
| 14. | >40 | 0.015 | 0.035 | 0.0065 | 0.05 | 0.26 |
| 15. | 0.004* | 0.24 | 7 | 0.48 | 3 | 16.4 |
| 16. | 0.004* | 1.9 | 1.7 | 0.485 | 1.9 | 5.5 |
| 17. | 0.004* | 2.1 | 1.3 | 0.25 | 1.1 | 1.3 |

* shows irreversible inhibition

Referring to Table 1, the chief way of identifying a beta-lactamase is by the widely different RSAI values given by different beta-lactamases for the same beta-lactam. Each RSAI value in the Table should be given at least a 20% margin for possible error. Normally there should be a factor of at least 2:1, between the RSAI values of beta-lactamases to be distinguished, and most preferably at least 10:1. The greater the difference in RSAI values the better. While some beta-lactamases are difficult to distinguish and on occasion might require the use of a beta-lactam giving RSAI's differing by a factor of only 2.5:1 or 4:1, up to 10:1, a ratio of 10:1 or higher is achievable in most instances. For example, cefoxitin gives RSAI=600 for SHV-1 but a value of no more than 60 for any of the other 10 commonly occurring beta-lactamases. A single assay therefore serves to make the distinction, thereby identifying or helping to identify SHV-1.

Further assistance in the identification is provided by the detection of irreversible inhibition. This term will now be explained. In the assay, an enzyme-substrate complex is formed as the reaction proceeds. The substrate can attach itself reversibly to the enzyme or become bound irreversibly. When reversible attachment occurs the beta-lactam ring is cleaved and the hydrolysis (cleavage) product is released rapidly from the substrate. Nitrocefin and many cephalosporin and penicillin antibiotics behave in this way towards most beta-lactamases. A plot of optical density (a measure of concentration of chromogenic substrate) against time is linear. More rarely, irreversible binding occurs, so the beta-lactam substrate is not released rapidly from the enzyme. (Occasionally it may become bonded to it covalently). After a short time, therefore, the number of active enzyme molecules becomes very small. A plot of optical density against time is therefore non-linear. A given competing beta-lactam substrate which shows irreversible binding does not do so towards every beta-lactamase. Consequently, the nature of the binding is an additional feature in the compilation of relative affinities, serving to help to identify a beta-lactamase. For example, clavulanic acid binds irreversibly to PSE-4 but reversibly to PSE-1, and is therefore particularly useful for distinguishing these two beta-lactamases.

Referring now to the commonly occurring beta-lactamases (Nos. 1—7 and 10—13 of the Table) the competing substrates can be selected on the basis of various protocols. One preferred protocol involves selecting competing substrates each of which will distinguish between the beta-lactamases or groups of beta-lactamases specified in, at least two and preferably all seven of the following pairings (1) to (7):—

(1) The group consisting of TEM-1, TEM-2 and SHV-1 from the group consisting of HMS-1, OXA-1, OXA-2, OXA-3, PSE-1, PSE-2, PSE-3 and PSE-4. Suitable beta-lactams include cefoxitin, moxalactam, R0-13-9904, cefotaxime and ceftizoxime, of which the first two are the most preferred.

(2) SHV-1 from the group consisting of TEM-1 and TEM-2. Suitable beta-lactams include cefoxitin, moxalactam, R0-13-9904 and MK 0787.

(3) The group consisting of PSE-1, PSE-2, PSE-3 and PSE-4 from the group consisting of HMS-1, OXA-1, OXA-2, and OXA-3. Suitable beta-lactams include SQ. 14,359, moxalactam and MK 0787.

(4) The group consisting of PSE-2 and PSE-3 from the group consisting of PSE-1 and PSE-4. Suitable beta-lactams include cefamandole, cepharadine, CP 45,899, clavulanic acid and MK 0787.

(5) PSE-2 from PSE-3. Suitable beta-lactams include cloxacillin, cephaloridine, cefotaxime, SQ 14,359, cefamandole, mecillinam R0-13-9904 and CP 45,899.

(6) HMS-1 from the group consisting of OXA-1, OXA-2 and OXA-3. Suitable beta-lactams include cefoperazone, carbenicillin, mecillinam, ceftazidime and MK 0787.

(7) OXA-1 from the group consisting of OXA-2 and OXA-3. Suitable beta-lactams include cephaloridine, cefoxitin, SQ. 14,359, CP 45,899, clavulanic acid, MK 0787 and ticarcillin.

The above protocol can be modified. One modification of possible advantage is to make pairing (3) effective to distinguish (3A), the group consisting of PSE-1, PSE-3 and PSE-4 from the group consisting of PSE-2, HMS-1, OXA-1, OXA-2 and OXA-3, and to set up a new pairing (3B) in which PSE-2 is distinguished from the group consisting of HMS-1, OXA-1, OXA-2, and OXA-3. PSE-2 is then omitted from pairing (4), which operates to distinguish PSE-3 over PSE-1 and 4. Pairing (5), PSE-2 from 3, is then omitted. Cefotaxime can then be used advantageously in (3A) to follow this protocol, while (3B) is met by cefamandole, cephradine, CP 45,899, clavulanic acid or MK 0787.

Similarly it can be advantageous to modify pairing (1) into a new pairing (1A) by making it inessential to distinguish the TEM and SHV-1 groups over OXA-1. In that case, OXA-1 is distinguished over TEM-1, TEM-2 and SHV-1 in a new pairing (1B). OXA-1 is then omitted from pairing (3), or (3A) and (3B) if the above modification is also applied, and also from pairing (6). Pairing (7) is omitted. A preferred substrate for (1A) is SQ 14,359 and preferred substrates for (1B) are moxalactam, cepharadine, mecillinam, ceftizoxime, R0-13-8804, CP 45,899 and MK 0787.

Since the same beta-lactams are often used for several of the 7 pairings, the number required in a kit for use with the assay is normally less than 7 and is usually from 2 to 5. Of course any number of further competing substates could be made available in a kit, for reinforcing a distinction. Thus, further substrates are preferably selected from one or more of the following further classes:—

(8) cloxacillin, cefotaxime, SQ 14,359, moxalactam, carbenicillin, ceftazidime, clavulanic acid, amoxycillin and ticarcillin;

(9) ampicillin, cloxacillin, cefoxitin and cefuroxime; and

(10) ampicillin, R0-13-9904, CP 45,889 and clavulanic acid.

The following are examples of preferred sets of substrates:—

(i) cefoxitin, (ii) moxaclactam or MK 0787 and (iii) mecillinam;

(i) cefoxitin, (ii) MK 0787 and (iii) any one of cloxacillin, cephaloridine, cefotaxime, SQ 14,359, cefamandole, R0-13-9904 and CP 45,899.

(i) cefoxitin, (ii) SQ 14,359, (iii) any one of cefamandole, cephradine, CP 45,899 clavulanic acid and MK 0787, and (iv) any one of cefoperazone, carbencillin, mecillinam and ceftazidime;

(i) cefoxitin (ii) cefotaxime and (iii) MK 0787;

(i) cefoxitin, (ii) cefotaxime, (iii) any one of cefamandole, cephradine, CP 45,899, and clavulanic acid and (iv) any one of cefoperazone, carbenicillin, mecillinam and ceftazidime.

(i) moxalactam, (ii) MK 0787 and (iii) any one of cloxacillin, cephaloridine, cefotaxime, SQ 14,359, cefamandole, mecillinam, R0-13-9904 and CP 45,899.

(i) moxalactam, (ii) CP 45,899 and (iii) any one of cefoperazone, carbenicillin, mecillinam and ceftazidime.

(i) moxalactam, (ii) mecillinam and (iii) clavulanic acid.

(i) R0-13-9904 and (ii) MK 0787.

(i) R0-13-9904, (ii) SQ 14,359, (iii) any one of cefamandole, cephradine, CP 45,899 and clavulanic acid and (iv) any one of cefoperazone, carbenicillin, mecillinam and ceftazidime.

If it is desired to distinguish between PSE-1 and PSE-4, clavulanic acid, CP 45,899 R0-13-9904 or ampicillin are suggested as a further beta-lactam.

When cefoxitin is used as the first beta-lactam, OXA-2 is already distinguished from OXA-3. Otherwise, cefoxitin, cloxacillin, cefuroxime or ampicillin are suggested when this distinction is desired.

All the above combinations can be supplemented or reinforced by using additional competing substrates. The Table of RSAI values will suggest further alternatives. Other alternatives will become available as new beta-lactams become available. These could be cephalosporins, thienamycins or monobactams, for example. The assays of the invention require only small quantities of the competing

substrate and, of course, since their use is purely diagnostic, even experimental beta-lactams which have not been clinically tested could be used.

It is not always necessary to distinguish every pair of candidate beta-lactamases. Indeed, it is a special advantage of the invention that it mimics a therapy situation. If a pair of candidate beta-lactamases is not readily distinguishable, it is virtually certain that there is no need to distinguish them, for the same therapy will probably apply to both. TEM-1 and TEM-2, OXA-2 and OXA-3 and PSE-1 and PSE-4 are the most difficult pairs of the commonly occurring beta-lactamases to distinguish. TEM-1 and TEM-2 have been shown to be virtually identical molecules, differing by a single amino acid and react very similarly towards beta-lactams. The invention is, of course, applicable to identifying a beta-lactamase uniquely or as a member of a small group of similar beta-lactamases, or merely for distinguishing two beta-lactamases or groups of beta-lactamases from each other. Thus, competing beta-lactams in the above lists of preferences could be omitted from the above lists where the distinction required is cruder or the number of candidate beta-lactamases smaller.

Considering the rarer of the 17 enzymes listed, the chromosomally-coded enzymes are readily distinguished as a group from all the others by moxalactam, cloxacillin, cefotaxime, SQ 14,359, amoxycillin, carbenicillin, ticarcillin, ceftazidime or clavulanic acid, for example. Several beta-lactams will distinguish the *Pseudomonas* chromosomally-coded enzyme from the *E. cloacae* one, including moxalactam, carbenicillin, cefamandole, mecillinam, ceftizoxime and R0-13-9904. *E. coli* SNU 35 has no significance at present, but is readily distinguishable from the *E. cloacae* enzyme by cloxacillin, R0-13-9904, cefamandole, cephaloridine, or ampicillin, for example.

SHV-1 and the Klebsiella enzymes K10317 and K1073 appear to be related. Antibodies to SHV-1 cross-react with K10317 and K1073 and their RSAI values are in general similar. It is therefore probably unnecessary to distinguish between them. If necessary, moxalactam or cloxacillin could be used to do so. The Klebsiella enzyme K-17 can usually be distinguished from all others in the Table by cefoxitin, but a confirmatory assay with cefoperazone or mecillinam is possibly useful.

The method of the invention can be used to identify a beta-lactamase which does not correspond to any known such enzyme or the basis of other characterising techniques. The method imparts to it a unique "fingerprinting".

In the quantitative method the relative affinities are preferably compiled as a table on paper or as part of a computer programme which can take any conventional form, for example magnetic tape. Preferably a computer is programmed to compute the relative affinities determined from the assay and to compare the determined relative affinities with those from the compilation.

In the "visual", i.e. the second main embodiment of the method of the invention, a different kind of relative affinity is used and the assay method also differs. Here, one starts by determining a concentration of competing beta-lactam substrate relative to chromogenic substrate at which differences between relative affinities will be recognisable visually when the competitive assays are carried out. In the assays, each competing beta-lactam substrate is used only at its predetermined concentration. By visual recognition it is then possible to determine roughly the degree of relative affinity in each assay and thereby identify the beta-lactamase.

The visual method is more easily understood by considering a specific case. An unknown beta-lactamase might be SHV-1 or OXA-1. Table 1 shows that cefoxitin gives a nitrocefin RSAI value of 600 with SHV-1 but only 20 with OXA-1. The RSAI value is determined at 50% inhibition of the chromogenic substrate, in this case nitrocefin. A suitable ratio of cefoxitin to nitrocefin to use in the visual method would therefore be, for example, 200:1. At this concentration the cefoxitin will inhibit the nitrocefin hydrolysis virtually completely when OXA-1 is the enzyme, but will compete only moderately when SHV-1 is the enzyme. Therefore a yellow colour will indicate that the beta-lactamase is OXA-1 and a reddish colour SHV-1. To take another example, moxalactam has nitrocefin RSAI values of 0.0001, 0.002 and 0.006 for the three chromosomally-coded enzymes in Table 1 and no other enzyme gives a value below 0.3 and most are in the range 1 to 100. Using a predetermined ratio of moxalactam to nitrocefin of 0.01:1, all enzymes except the chromosomally-coded ones will give a positive result (red color) in the assay.

As will be evident from the above illustrations, the visual method works best by "picking off" the beta-lactamases which have either exceptionally low or exceptionally high RSAI values by comparison with other beta-lactamases from which the test is required to distinguish. It can be used to distinguish between a pair of beta-lactamases, to identify a beta-lactamase as an individual from a small or large group or to identify a beta-lactamase as a member of a small group.

The compilation of relative affinities in the visual method is preferably based on a simple scoring system involving at most 3 kinds of score, according to whether there is substantially no spectral change, a small change or a large one. For nitrocefin, the preferred scoring system is therefore very red, red or yellow. It can take the form of a table and/or colour chart, for example. Sometimes the borderline between very red and red causes difficulty and a colour chart is particularly helpful here. Obviously it is desirable to arrange that the enzymes to be distinguished give a score which is either strongly positive (very red) or negative (yellow).

Generally stated, the same protocol as set out above for the quantitative method applies to the visual method. Of course the choice of beta-lactams is more restricted if no spectrophotometer is used since it is difficult to detect irreversible inhibition by eye. Commonly occurring beta-lactamases can readily be

identified as (1) SHV-1, (2) TEM-1 or TEM-2, (3) HMS-1, (4) OXA-1, (5) OXA-2 or OXA-3, (6) PSE-1 or PSE-4, (7) PSE-2 or (8) PSE-3 by carrying out five assays, using nitrocefin and the following competing beta-lactams: cefoxitin at a high concentration sufficient to distinguish SHV-1 from all the other listed enzymes; cefotaxime at high concentration sufficient to distinguish (a) PSE-3 from PSE-2 and (b) TEM-1, TEM-2 and SHV-1 from HMS-1 and OXA-1; cefamandole, cephradine or MK 0787 at a concentration which distinguishes the PSE-1 and 4 group from the PSE-2 and 3 group; mecillinam at a concentration which distinguishes HMS-1 and OXA-1 and cefoxitin at a lower concentration than above, sufficient to distinguish the group TEM-1, TEM-2, SHV-1, HMS-1 and OXA-1 from OXA-2 and OXA-3 and preferably also from the group PSE-2, 3 and 4. Example 8 illustrates this method, using a 6-well plate. If desired a nitrocefin control can be used in the sixth well or it might be preferred to screen the sample first, e.g. with nitrocefin, to detect whether any kind of beta-lactamase is present. Alternative beta-lactams will be found listed in Example 9 which includes the first 4 beta-lactam tests as in Example 8. In each case it is to be understood that concentrations of competing beta-lactam substrate to nitrocefin are not limited rigidly to those shown in Examples 8 and 9, but can be varied to make the desired visual distinctions.

The scores in the visual tests do not necessarily correlate completely with the RSAI values in Table 1. This is primarily because the Table 1 values relate to assays carried out at 37°C, while the visual tests are sometimes carried out at room temperature. Obviously, too drastic a variation in temperature will affect the relative affinity, since beta-lactamases differ in their temperature-reaction rate relationships. It is of course necessary to use a compilation of relative affinities which holds good for the temperature at which the assays are to be carried out.

If one of the chromosomally-coded enzymes is present in the sample further competitive assay(s) will identify them more precisely. For example, several beta-lactams will distinguish the *Pseudomonas aeruginosa* chromosomally-coded enzyme from the other two, e.g. mecillinam, R0-13-9904 or ceftizoxime. For visual differentiation, a mecillinam to nitrocefin ratio of about 20:1 is suggested. This will inhibit the *E. cloacae* and *E. coli* enzymes without affecting the *Ps. aeruginosa* enzyme. *E. coli* SNU 35 is a genetically engineered enzyme which at present has no clinical or natural significance and therefore further assays are not needed. If, however, it should arise through induction, it is readily distinguishible from the *E. cloacae* enzyme by an assay using R0-13-9904, e.g. at a ratio to nitrocefin of about 0.001:1.

In a more sophisticated form the above well-plate method can be expanded so as to identify each of the 17 beta-lactamases set out in Table 1. Spectrophotometric scanning at a wavelength of 486 nm of the wells, incubated at 37°C for the assay, allows the detection of irreversible binding of the competing substrate. This is essential to distinguish K10317 from K1073 and helps to distinguish PSE-1 from PSE-4 using moxalactam or cefoxitin as competing substrates for the former and clavulanic acid for the latter pair of beta-lactamases. This "visual-spectrophotometric" embodiment of the visual method is illustrated in Example 9, in which up to 11 wells of an 8×12 microtitre plate are used for each assay.

The visual and quantitative methods can, of course, be used in sequence if desired, the simple visual method with monitoring merely by eye being used for the routine identifications. Any sample which gives an ambiguous answer in the simple visual assay is put through a full "visual-spectrophotometric" or quantitative assay. This technique is particularly useful if a bacterial extract produces a mixture of beta-lactamases, if one of the rarer beta-lactamases is suspected or if it is desired to distinguish between PSE-1 and 4, for example.

Other techniques are usable in reducing the number of candidates to be distinguished by the visual method. For example, isoelectric focussing could be used. The test of the invention is a valuable adjunct to isoelectric focussing since many beta-lactamases have very similar isoelectric points and cannot be distinguished by isoelectric focussing alone.

The assay can be carried out in any manner in which competition is produced between the chromogenic substrate and the other beta-lactam substrate. Clearly, neither substrate must become exhausted during the assay and sufficient concentrations must be present to provide an excess of total substrate with respect to the capacity of the enzyme.

Where an irreversibly binding beta-lactam substrate is used, it is possible to "pre-incubate" the substrate with the enzyme and then subsequently add a reversibly attaching chromogenic substrate preferably nitrocefin. Of course, in the pre-incubation (preliminary reaction) stage, one must not add so much substrate that all available enzyme sites are consumed. RSAI values obtained after pre-incubation will differ from those obtained when the nitrocefin is added initially only if irreversible binding has occurred (see Example 3).

There are various ways of proceeding when two or more beta-lactam substrates are to be used for identification of the beta-lactamase. In one such way, the second and any subsequent beta-lactam substrate is chosen after considering the result of the previous assay. In other words, the choice of the second standard substrate would depend on the particular possibilities present when the relative affinity of the first standard substrate was determined, and, likewise, if a third standard substrate were needed, it would be chosen according to the affinity results for the first two.

In a preferred way of proceeding, the second and a sufficient number of subsequent competing substrates are chosen in advance of the first assay, so that any unidentified beta-lactamase is identifiable from the assays. In this way of proceeding, it is contemplated that a supply of, say, 4, 5, 6 or 7 competing substrates would be as many as is normally required to test for all the common beta-lactamase-producing

bacteria. One might wish to use more substrates, for example as many as 10 for distinguishing rarer beta-lactamases as well as the commoner ones.

The invention provides a kit for use in the quantitative method of the invention which comprises at least five sets of mixtures of the chromogenic substrate and a competing substrate, each in at least 4 different ratios per set and the competing substrate being different in each set of mixtures, and the chromogenic substrate *per se*, as a control.

Preferably the kit also includes (c) a member carrying a compilation of relative affinities and preferably also (d) one or more identified beta-lactamases (which term includes beta-lactamase-producing bacterial cultures or culture extracts), to serve as controls for standardising or testing the assay. The kit could comprise pre-prepared units, e.g. impregnated absorbent discs, each containing a mixture of known quantities of (i) one or more of the competing substrates with (ii) the chromogenic substrate. A supply of the chromogenic substrate alone forms part of the kit. Another possible form of the kit is pre-prepared microtitre plates in which the mixture of competing and chromogenic substrate is placed in the wells.

For the visual method, the chromogenic and competing substrates are used in a fixed ratio in each assay although the same competing substrate might be used in a different ratio to the chromogenic substrate in two different assays. Therefore for this purpose the invention includes a kit which comprises:—

(a) at least five sets of a mixture of the chromogenic substrate with a competing substrate, wherein the competing substrate is the same or different between one set and another, but, if the same, is present in no more than two sets in different ratios, each mixture being such that a visually recognisable difference in the spectrum of the chromogenic substrate would occur between competitive assays of different beta-lactamases using the mixture and

(b) the chromogenic substrate *per se*, as a control.

Again, the kit can include an identified beta-lactamase for testing the kit.

Whether the competing substrates are supplied *per se* or in a mixture with e.g. nitrocefin, the substrates supplied will preferably be any of those which are effective to make all the distinctions in pairings 1 to 7 at least, and can be any of the sets of beta-lactams referred to above. They can be any of the specific sets or "combinations" of beta-lactams set out above, omitting, of course, from consideration for the simple visual method those which depend on irreversible inhibition to make the particular distinction required. For the simple visual method cefoxitin will nearly always be supplied, preferably together with (2) cefotaxime, (3) cefamandole, cephradine or MK 0787, and (4) mecillinam. In the quantitative method cefoxitin, moxalactam, MK 0787, mecillinam, CP-45,899 and possibly cloxacillin are one valuable basis for a kit.

The invention is very useful in identifying beta-lactamases present in isolates obtained directly from patients, especially in samples of human blood. Patients suffering from a severe infection caused by gram negative bacteria are usually best treated by beta-lactam therapy. When a patient fails to respond quickly to a beta-lactam antibiotic, it is often necessary, very urgently, to discover whether he is producing a beta-lactamase which is destroying that antibiotic. More practically, the object is to find out which antibiotics are likely to be successful in treating the patient. At present the hospital laboratory tests the isolate by sub-culturing the bacteria on discs of a few candidate antibiotics, hoping that the bacteria will die on some of the antibiotics discs. The method takes routinely at least 24 hours from the beginning of the sub-culture, by which time the patient's condition might have deteriorated irretrievably. It is limited as to the number of candidate antibiotics and by the use of a single concentration of antibiotic. The use of several different concentrations of each antibiotic, in so-called "break-point" tests, ho determine the concentration which will inhibit growth of the culture, is better but still takes at least 24 hours.

The present invention does not require a sub-culture step and is therefore very much more rapid. It is possible to carry it out easily within one hour from the production of a primary culture and therefore can be used routinely to give results during the same working day. Further, the method of the invention has a built-in adaptability, because it yields information about the best antibiotics to use to counter an infection caused by any beta-lactamase-producing bacterium. This means that it can be used to suggest treatment for infections caused by rare or even novel beta-lactamases. A high RSAI value, for example, indicates that an antibiotic is likely to be effective against that particular beta-lactamase. It is also possible to suggest combination therapy of the "Augmentin" type, using an irreversible inhibitor to tie up the beta-lactamase followed by a beta-lactam antibiotic.

The method of the invention is also very useful in epidemiology. When an epidemic occurs in a hospital considerable detection is often needed to find the source or sources of infection. The first steps in the detection operation would involve determining how many patients, and in which wards, are suffering from infection by the same organism. Because the assay of the invention is so quick to perform and reliable, it is possible to identify beta-lactamases from, say, 100 bacterial samples and thereby identify the extent to which a single organism is responsible for the epidemic.

Another use of the invention is in environmental health, for identifying bacteria found in food or drink or places where food or drink is stored, displayed or consumed.

The clinically significant beta-lactamases present in most bacteria are constitutive and are located within the cell. A very few bacteria produce extracellular beta-lactamase, but usually the quantities involved are small. Normally, therefore, the beta-lactamase has to be extracted from the cells for the

purpose of the present invention. One preferred method comprises digesting a suspension of a bacterial cell culture with a cell wall-weakening agent, preferably lysozyme, and subjecting the suspension to successive freezing and thawing and recovering liquid from the product, preferably by centrifuging and drawing off supernatant liquid. It has been found that a single freezing and thawing cycle suffices for ordinary bacterial cells. The freezing is preferably to a temperature of from −40°C to −78°C, most usually about −70°C. Example 5 illustrates the procedure. The total time required for an assay using this method of extracting the beta-lactamase is 4—5 hours, compared with 36 hours or so using a conventional method of extraction. Example 6 describes an even more preferred method, currently considered to be the best mode of extraction for the purposes of this invention. This method takes about 20 minutes.

Other methods of extracting beta-lactamase from bacterial cells are described in Example 1 and in the paper by Simpson *et al*, Antimicrobial Agents and Chemotherapy, *17*, 929 (1980).

The invention is illustrated by the following examples which refer to the accompanying drawings. In the drawings:—

Figure 1 is a graph showing optical density plotted against time for the reaction of nitrocefin alone with TEM-2 beta-lactamase enzyme, using a series of different concentrations of nitrocefin;

Figure 2 is a "double reciprocal plot" (Lineweaver-Burk) graph using the results from Figure 1, in which the reciprocal of reaction velocity is plotted against the reciprocal of the concentration of nitrocefin;

Figure 3 is a graph showing optical density plotted against time for assays of the invention using nitrocefin, TEM-2 and various different concentrations of antibiotic ampicillin;

Figure 4 is a graph using the results from Figure 3, in which the % hydrolysis of nitrocefin after 4.5 minutes is plotted against the concentration of antibiotic;

Figure 5 is a graph showing optical density plotted against time for assays of the invention using nitrocefin, TEM-2 and different concentrations of the antibiotic cefoxitin;

Figure 6 is a graph showing optical density plotted against time for assays for the invention using nitrocefin, TEM-2 and different concentrations of the beta-lactamase irreversibly binding substrate clavulanic acid;

Figure 7 is two graphs drawn on the same co-ordinates, the left-hand graph being a repeat of Figure 6 and the right-hand one a corresponding graph for an assay in which the TEM-2 enzyme was pre-incubated with the beta-lactam;

Figure 8 is a graph using the results from Figure 7 in which the % hydrolysis of nitrocefin after 4.5 minutes is plotted against the concentration of clavulanic acid; and

Figure 9 is a graph similar to Figure 8 but with the antibiotic cloxacillin in place of clavulanic acid.

Example 1

Preparation of beta-lactamases

*E. coli* C9 was obtained from the *E. coli* Genetic Stock Centre, New Haven, Connecticut. The transmissible plasmid RP4 which codes for the TEM-2 beta-lactamase, Datta et al., J. Bacteriol. *108*, 1244—1249 (1971), was transferred into *E. coli* C9 by plate-mating. For the preparation of beta-lactamase extract 1 litre of *E. coli* C9 RP4 was grown to a stationary phase in Oxoid Nutrient broth medium at 37°C with shaking, in a 3 litre flask. The periplasmic proteins of *E. coli* C9 RP4 were prepared from the culture using the tris-EDTA extraction method of Levy & Leive, J. Biol. *Chem.* 245,548, (1970). From 1 litre of cells 7 ml of tris-EDTA periplasmic extract was obtained. This contained the TEM-2 beta-lactamase enzyme coded for by the plasmid RP4. For routine assay much smaller volumes of culture will be used.

Hydrolysis of nitrocefin by enzyme

The following mixture was prepared in a 1 cm light path spectrophotometer cuvette; 890 microlitres of 10 mM sodium phosphate buffer of pH 7.0 prewarmed to 37°C, and 100 microlitres of nitrocefin made up in 10 mM phosphate buffer of pH 7.0 at 500 micrograms/ml. After temperature equilibration to 37°C in the heated cuvette holder, 10 microlitres of a 1 in 10 dilution of the tris-EDTA periplasmic extract (diluted in 10 mM phosphate buffer of pH 7.0) was added. The change in optical density at a wavelength of 486 nm was recorded over a 5 minute period. The reference cuvette contained buffer and nitrocefin but not enzyme.

Assay

For each competition assay, a known amount of a beta-lactam made up to 100 microlitres in 10 mM phosphate buffer pH 7.0, was substituted for an equivalent volume of buffer in the assay mixture. For each beta-lactam a series of assays (at least 4) was performed, using a different concentration.

Results

Kinetics of nitrocefin cleavage by TEM-2 beta lactamase

The effect of varying the concentration of nitrocefin ("NCF") on the optical density at 486 nm is shown in Figure 1. A double reciprocal plot of this data is shown in Figure 2. The ordinate represents the reciprocal of reaction velocity (optical density at 486 nm after 3 minutes reaction time). The abscissa represents the reciprocal of the initial concentration of nitrocefin (enzyme substrate) in mg/ml. The Michaelis constant ($K_m$) of the enzyme with respect to nitrocefin was calculated from the plot as 25 micrograms/ml. In the assays, 50 micrograms/ml of nitrocefin was used in the assay mixture. That the beta-lactamase enzyme TEM-2, coded

for by the RP4 plasmid, was present was confirmed by substituting in the assay 10 microlitres of undiluted, or a 1 in 10 dilution, of the tris-EDTA periplasmic extract of *E. coli* C9; there was no change in the optical density at 486 nm during the time of the assay with this preparation.

Assay for beta-lactam antibiotics

Beta-lactam antibiotics which bind to the TEM-2 enzyme compete with nitrocefin in the assay. The effect of varying concentrations of ampicillin ("AMP") on the cleavage (hydrolysis) of nitrocefin is shown in Figure 3. From a plot of nitrocefin cleavage against concentration of ampicillin one can determine the ampicillin concentration which results in 50% inhibition of the TEM-2 enzyme activity (Figure 4). This concentration of ampicillin is a direct estimate of the relative affinity of the TEM-2 enzyme for ampicillin. The Relative Substrate Affinity Index (RSAI), which is the ratio of the concentration of beta-lactam giving 50% inhibition of nitrocefin cleavage (65 micrograms/ml) to the concentration of nitrocefin used in the assay (50 migrograms/ml) was calculated. The RSAI value was therefore 1.3.

Example 2

The effect of competition by beta-lactam substrates on the kinetics of nitrocefin cleavage also yields information on the mechanism of interaction between the antibiotic and the beta-lactamase. Most of the beta-lactam substrates tested against the TEM-2 enzyme show a similar pattern of inhibition to that of ampicillin, i.e. increasing the concentration of beta-lactam reduces the rate of nitrocefin cleavage (shown as a reduced slope of the optical density value) but the plots are still linear, as shown in Figure 3. This is characteristic of competitive inhibition. In contrast when cefoxitin ("CEF") was substituted for ampicillin, it was found that the rate of cleavage (hydrolysis) of nitrocefin diminished with time. This is shown in Figure 5. It indicates that cefoxitin undergoes irreversible binding to the enzyme.

Example 3

Example 2 was repeated, substituting clavulanic acid for cefoxitin. Similar kinetics were also obtained with clavulanic acid, although at much lower concentrations of clavulanic acid (see Figure 6).

The assay was repeated, with the variation that the enzyme was pre-incubated with clavulanic acid for 5 minutes at 37°C. This resulted in a drastic reduction in the rate of cleavage of the nitrocefin substrate. This is shown in Figure 7 (compare especially the plot for 100 ng/ml of clavulanic acid (a) at the left-hand side, where there was no pre-incubation and (b) at the right-hand side, with pre-incubation).

In Figure 8, % nitrocefin cleavage is plotted against clavulanic acid concentration for the assays without (A) and with (B) pre-incubation. The % nitrocefin cleavage is taken from the values in Figure 7 after 4.5 minutes. It will be seen that the respective RSAI values are (A) 0.005 and (B) 0.0004.

Example 4

Example 3 was repeated using cloxacillin in place of clavulanic acid. The results are plotted in Figure 9 similarly to Figure 8, curve A representing the ordinary assay and B the assay with the 5 minute pre-incubation. There is little difference in the two curves. Cloxacillin clearly does not undergo irreversible binding.

Example 5

This example describes the preferred, freeze-thaw/weaken cell walls, technique of extracting beta-lactamase from cells. A culture of the bacterium (*E. coli* C9 RP4) was grown at 37°C in nutrient broth. One ml volumes of the culture were centrifuged and the supernatant discarded. The pellet remaining was emulsified by whirlmixing and then 100 microlitres of a solution containing 400 micrograms of lysozyme and 50 mM in glucose, 10 mM in cyclohexane diamine tetraacetate and 5 mM in Tris-HCl, to give pH 8.0, was added to the suspension. The digestion with the lysozyme solution was allowed to proceed at 4°C for 30 minutes. The suspension was then frozen at −70°C for 10 minutes and thawed at 37°C for 2 minutes. One freeze-thaw cycle was found to be sufficient. The debris was removed by centrifuging. The supernantant liquid contained the desired beta-lactamase enzyme.

The above technique was repeated for other bacteria, vis: *Klebsiella aerogenes, Klebsiella pneumoniae, Enterobacter cloacae*, and *Pseudomonas aeruginosa*.

Example 6

This example describes another preferred technique of extracting beta-lactamase from cells, based on the work of Choma and Yamazaki in the Canadian Journal of Microbiology *27*, 547 (1981). Cultures are centrifuged at 9,000 rpm for 10 minutes and the cell pellets resuspended in one tenth the original volume of a solution of 1% (weight/volume) phenethyl alcohol in 10 mM phosphate buffer at pH 7.0. After gentle shaking at 20°C for 10 minutes the cell suspension was spun at 9,000 rpm for 10 minutes. The supernatant liquid was removed. It contained more than 90% of the beta-lactamase in the original culture.

Example 7

This example described an actual identification of an "unknown" (unidentified) beta-lactamase. A culture of gram negative bacteria was isolated from a river. The cells of a sample were extracted by the

method of Example 6. The assay was carried out as described in Example 1 and gave RSAI values of 0.008 with cefoxitin and 0.0001* (*=showed irreversible inhibition) with moxalactam. Either result on its own indicates *Enterobacter cloacae*. Another sample of the culture was sent to a Public Health microbiology laboratory for independent identification. Their tests confirmed the identification.

Example 8

This example describes a visual test for identifying or assisting in the identification of the 11 commonly encountered beta-lactamases plus the chromosomally-coded *E. cloacae* and *Pseudomonas aeruginosa* ones. Nitrocefin (25 micrograms) was placed in six wells of a microtitre dish for each enzyme to be assayed. The sixth well is the control and competing beta-lactams at the concentrations shown in Table 2 below were placed in wells 1 to 5. Twenty microlitre volumes of enzyme extract were added to all six wells and the red colour developed scored after 3 minutes at room temperature (about 20°C). The scoring system was:—

++Intensity of red colour similar to the control
+Intensity of red colour significantly less than the control
−Little red colour developed

This sample rapid screening system is aided by colour photographs of the range of red colours associated with the scoring system. It is capable of automation with spectrophotometer scanning of the wells and microprocessor interpretation of the results to give a print out of the beta-lactamase identification. The use of sensitive spectrophotometer scanning instead of a visual estimation of the red colour widens the range of beta-lactams capable of being used to discriminate beta-lactamases, due to the extra sensitivity of detection.

This simple system positively identifies SHV-1, HMS-1, OXA-1, *E. cloacae*, PSE-2, and PSE-3. It does not discriminate between OXA-2, OXA-3 and *Pseudomonas aeruginosa* beta-lactamases although it does identify them from all the others. Similarly it does not discriminate PSE-1 from PSE-4. However, this is possible using the more sensitive detection offered by spectrophotometer scanning or by the more sophisticated test in up to 11 wells described in Example 9. It is not possible or necessary to discriminate the virtually identical TEM-1 and TEM-2 enzymes.

TABLE 2: Scores in 6-well visual test

| Well | Beta-lactam | |
|---|---|---|
| A | = Cefoxitin 20:1 | (Ratio to nitrocefin) |
| B | = Cefotaxime 100:1 | |
| C | = Cefamandole 1:1 | ++=Very red |
| D | = Mecillinam 20:1 | +=Red |
| E | = Cefoxitin 200:1 | −=Yellow |

| Enzyme | A | B | C | D | E |
|---|---|---|---|---|---|
| 1. TEM-1 | ++ | ++ | ++ | ++ | − |
| 2. TEM-2 | ++ | ++ | ++ | ++ | − |
| 3. SHV-1 | ++ | ++ | ++ | ++ | + |
| 4. HMS-1 | ++ | − | + | − | − |
| 5. OXA-1 | ++ | − | ++ | ++ | − |
| 6. OXA-2 | − | − | + | + | − |
| 7. OXA-3 | − | − | + | + | − |
| 8. *E. cloacae* | − | − | − | − | − |
| 10. PSE-1 | ++ | + | ++ | ++ | − |
| 11. PSE-2 | + | − | − | ++ | − |
| 12. PSE-3 | + | + | − | + | − |
| 13. PSE-4 | + | + | ++ | ++ | − |
| 14. Ps+AMP (Chromosomal) | − | − | + | + | − |

Example 9

This example illustrates the "visual-spectrophotometric" method, suitable for use in an 8×12 well microtitre plate. It includes the first 4 tests (A-D) as described in Example 8, a different test (E), plus up to 5 optional further tests. A control well (K) containing nitrocefin alone is used when no pre-screening is done. The assay conditions are as described in Example 8, except that the temperature is 37°C, and spectrophotometric scanning at 486 nm, is used to detect irreversible inhibition. Table 3 shows one form of the method. Notes on Table 3 suggest alternative substrates and Table 4 shows a method using these alternatives. In each case, the ratios used or suggested are approximate guides only and can be varied considerably depending, for example, on the temperature used for assay, and whether the protocol of distinguishing is varied.

TABLE 3: Scores in an 11-well visual assay carried out with the aid of spectrophotometric scanning to detect irreversible inhibition (*)

| Well | Beta-lactam | |
|---|---|---|
| A | = Cefoxitin 20:1 | (Ratio to nitrocefin) |
| B | = Cefotaxime 100:1 | |
| C | = Cefamandole 1:1 | |
| D | = Mecillinam 20:1 | ++=Very red |
| E | = Moxalactam 100:1 | +=Red |
| F | = Moxalactam 0.01:1 | −=Yellow |
| G | = Cloxacillin 1:1 | *=Irreversible inhibition |
| H | = Clavulanic acid 0.004:1 | |
| I | = Cefoxitin 200:1 | |
| J | = R0-13-9904 0.001:1 | |

| Enzyme | A | B | C | D | E | F | G | H | I | J |
|---|---|---|---|---|---|---|---|---|---|---|
| 1. TEM-1 | ++ | ++ | ++ | ++ | +* | ++ | + | +* | +* | ++ |
| 2. TEM-2 | ++ | ++ | ++ | ++ | +* | ++ | + | +* | +* | ++ |
| 3. SHV-1 | ++ | ++ | ++ | ++ | ++ | ++ | ++ | +* | ++ | ++ |
| 4. HMS-1 | + | – | + | – | – | + | – | +* | + | ++ |
| 5. OXA-1 | + | – | + | ++ | – | ++ | + | ++* | + | ++ |
| 6. OXA-2 | – | – | + | ++ | – | ++ | ++ | ++* | – | ++ |
| 7. OXA-3 | – | – | + | ++ | – | ++ | – | ++* | – | ++ |
| 8. *E. cloacae* | – | – | – | – | – | – | – | ++ | – | – |
| 9. SNU 35 | – | – | – | – | – | – | – | ++ | – | ++ |
| 10. PSE-1 | + | + | ++ | ++ | – | ++ | + | – | – | ++ |
| 11. PSE-2 | + | – | – | ++ | – | ++ | ++ | ++ | – | ++ |
| 12. PSE-3 | + | + | – | + | – | ++ | – | + | – | ++ |
| 13. PSE-4 | + | + | ++ | ++ | – | ++ | ++ | +* | – | ++ |
| 14. *Ps.* +AMP | – | – | ++ | ++ | – | – | – | ++ | – | ++ |
| 15. K10317 | ++ | ++ | ++ | ++ | +* | ++ | ++ | + | ++* | ++ |
| 16. K1073 | ++ | ++ | ++ | ++ | ++ | ++ | ++ | + | ++ | ++ |
| 17. K17 | – | + | + | ++ | – | ++ | ++ | + | – | ++ |

Notes on Table 3

Well A. Cefoxitin, together with cloxacillin in well G, distinguishes OXA-1 from OXA-2. Alternatives are SQ 14,359 10:1 or MK 0787 0.1:1.

Well B. Cefotaxime, together with cloxacillin in well G, helps to identify (1) PSE-2 and PSE-3. Cefotaxime also distinguishes (2) TEM-1, TEM-2, SHV-1, K10317 and K 1073 from all the others. Alternatives for (1) are CP 45,899 1:1 or mecillinam 200:1, and for (2) are moxalactam 20:1 or R0-13-9904 100:1.

Well C. Cefamandole distinguishes (1) PSE-1 and PSE-4 from PSE-2 and PSE-3 and (2) *E. cloacae* and SNU 35 from the *Ps. aerug.* chromosomal enzyme ("Ps. +AMP"). Alternatives for (1) are cephradine 20:1 or MK 0787 5:1 and for (2) mecillinam 20:1.

Well D. Mecillinam distinguishes (1) *E. cloacae* (and SNU 35) from Ps. +AMP. It also distinguishes very clearly (2) between HMS-1 and OXA-1. Alternatives for (1) are cefamandole 1:1 and for (2) ceftazidime 500:1.

Well E. Moxalactam at this high concentration distinguishes SHV-1 and K1073 from all the others, including K 10317.

Well F. Moxalactam at this low concentration identifies all the chromosomal enzymes. Alternatives are cloxacillin 0.001:1, cefotaxime 0.01:1 and SQ 14,359 0.01:1.

Well G. Cloxacillin distinguishes (1) OXA-2 from OXA-3, (2) PSE-2 from PSE-3 and (3) HMS-1 from OXA-1. Alternatives are (1) cefoxitin 0.01:1 or cefuroxime 0.1:1, (2) CP 45,899 1:1 or mecillinam 200:1 and (3) ceftazidime 250:1. As regards distinction (3) the well A test will often help, especially if SQ 14,359 is used.

Well H. Clavulanic acid distinguishes PSE-1 from PSE-4. CP 45,899 at 0.015:1 is an alternative.

Well I. Cefoxitin at a low concentration reinforces the identifications of SHV-1 and K 1073 given by well E.

Well J. R0-13-9904 distinguishes *E. cloacae* from SNU 35.

Each alternative does not necessarily perfectly replace the beta-lactam of Table 3, but all other beta-lactams being as in Table 3, will not confuse the identification which is the primary role of the

beta-lactam. However, most of the alternatives can be used together with any other alternative. This is illustrated by Table 4 where 7 of the 10 wells are occupied by alternatives. Combinations of alternatives can be worked out from RSAI values in Table 1.

TABLE 4: Scores in another 11-well visual assay carried out with the aid of spectrophotometric scanning to detect irreversible inhibition (*)

| Well | Beta-lactam | |
|---|---|---|
| A | = SQ 14,359 10:1 | (Ratio to nitrocefin) |
| B | = Moxalactam 20:1 | |
| C | = Cephradine 20:1 | |
| D | = Ceftazidime 500:1 | |
| E | = Moxalactam 100:1 | |
| F | = Cloxacillin 0.001:1 | |
| G | = Cefoxitin 0.01:1 | |
| H | = CP 45,899 1:1 | |
| I | = Clavulanic acid 0.004:1 | |
| J | = Cefoxitin 200:1 | |

| Enzyme | A | B | C | D | E | F | G | H | I | J |
|---|---|---|---|---|---|---|---|---|---|---|
| 1. TEM-1 | +* | ++* | ++ | ++ | +* | ++ | ++ | – | +* | +* |
| 2. TEM-2 | +* | ++ | ++ | ++ | +* | ++ | ++ | – | +* | +* |
| 3. SHV-1 | ++* | ++ | + | ++ | ++ | ++ | ++ | – | +* | ++ |
| 4. HMS-1 | – | – | + | – | – | + | ++ | – | +* | + |
| 5. OXA-1 | ++* | – | ++ | ++ | – | ++ | ++ | ++ | ++* | – |
| 6. OXA-2 | – | – | ++ | ++ | – | ++ | ++ | – | ++* | – |
| 7. OXA-3 | – | – | ++ | ++ | – | + | – | – | ++* | – |
| 8. *E. cloacae* | – | – | – | – | – | – | + | ++ | ++ | – |
| 9. SNU 35 | – | – | – | – | – | – | – | +* | ++ | – |
| 10. PSE-1 | – | – | ++ | + | – | ++ | ++ | – | – | – |
| 11. PSE-2 | + | + | – | + | – | ++ | ++ | ++ | ++ | – |
| 12. PSE-3 | – | + | – | + | – | ++ | ++ | – | + | – |
| 13. PSE-4 | – | – | ++ | + | – | ++ | ++ | – | +* | – |
| 14. *Ps.* +AMP | – | – | – | – | – | – | – | +* | ++ | – |
| 15. K10317 | ++ | + | + | ++ | +* | ++ | ++ | – | + | ++* |
| 16. K1073 | ++ | + | + | ++ | ++ | ++ | ++ | – | + | ++ |
| 17. K17 | + | – | + | ++ | – | ++ | ++ | + | + | – |

Example 10

This example illustrates use of an alternative chromogenic substrate "PADAC". "PADAC" in aqueous solution (10 mM phosphate buffer pH 7.0) is a deep violet colour absorbing strongly at 570 nm. On cleavage it is said to go yellow and generate a new absorption maximum at 465 nm. However, "PADAC" cleavage at 465 nm is very slow and cannot be monitored in a competition assay. On the other hand, using a 1 ml reaction volume of "PADAC" (50 micrograms), enzyme and buffer (10 mM phosphate pH 7.0), cleavage can readily be followed by monitoring the fall in absorption at 570 nm. The initial optical density at 570 nm was set at 1.0, the enzyme was added and the reaction was followed over a 3—4 minute period. The control incubation results in a fall in OD570 to about 0.1 during that time. The effect of competing beta-lactams was then performed as for nitrocefin, and the RSAI values determined. Only one beta-lactam, cefoxitin, was tested, this being sufficient to show that "PADAC" is usable but not as convenient as nitrocefin. The disadvantages of PADAC are:—

(a) there was no cleavage of "PADAC" by OXA-1, yet the same enzyme preparation cleaved nitrocefin;
(b) more enzyme has to be used than in the nitrocefin assay, i.e. PADAC is less sensitive; and
(c) The assay cannot be used in a simple visual test: a spectrophotometer is needed.

TABLE 5: RSAI values (PADAC)

| Beta-lactamase | Cefoxitin |
|---|---|
| 1. TEM-1 | 15* |
| 2. TEM-2 | 18* |
| 3. SHV-1 | 140 |
| 4. HMS-1 | 55 |
| 5. OXA-1 | No reaction |
| 6. OXA-2 | 0.1 |
| 7. OXA-3 | 0.00001 |
| 10. PSE-1 | 25 |
| 11. PSE-2 | 6 |
| 13. PSE-4 | 46 |
| 14. Ps +AMP | 0.00002 |
| 15. K10317 | 320 |
| 16. K1073 | 112 |
| 17. K17 | 0.8 |

**Claims**

1. A method of identifying an unidentified beta-lactamase, which method comprises performing a competitive assay on said beta-lactamase with (1) a chromogenic beta-lactam-containing substrate capable of undergoing a change in its visible light spectrum when reacted under the competitive assay conditions with beta-lactamases and (2) a competing substrate for the beta-lactamase, monitoring the assay for a change in the visible light spectrum of the chromogenic substrate, thence determining a relative affinity of the competing substrate to the chromogenic substrate for the unidentified beta-lactamase, repeating said assay at least four times using for each assay a different competing substrate or the same competing substrate in no more than two different ratios of the competing substrate to the chromogenic substrate and identifying the beta-lactamase by reference to a compilation of previously determined relative affinities of each competing substrate relative to the chromogenic substrate for different beta-lactamases.

2. A method according to Claim 1 wherein the chromogenic substrate is nitrocefin.

3. A method according to Claim 1 or 2 wherein the relative affinity is determined quantitatively and each assay is carried out in a manner which enables any irreversible inhibition of the beta-lactamase by the competing substrate to be detected.

4. A method according to Claim 3 using at least two different competing substrates wherein each

individual assay is carried out using at least 4 different ratios of competing substrate to chromogenic substrate and a relative affinity is determined as the ratio:—

$$\frac{\text{Concentration of beta-lactam-containing substrate which gives a defined percentage inhibition of cleavage of the chromogenic substrate}}{\text{Concentration of chromogenic substrate.}}$$

5. A method according to Claim 4 wherein the defined percentage is 50 per cent.

6. A method according to Claim 3, 4, or 5 wherein the assay is monitored spectrophotometrically.

7. A method according to Claim 6 in which the competing substrates will distinguish between the beta-lactamases or groups thereof by a ratio of at least 2.5:1 in their relative substrate affinity index defined by the ratio:—

$$\frac{\text{Concentration of beta-lactam-containing substrate which gives 50\% inhibition of cleavage of the chromogenic substrate.}}{\text{Concentration of the chromogenic substrate}}$$

or will distinguish them according to whether or not they give irreversible inhibition.

8. A method according to Claim 7 wherein the distinguishing ratio is at least 10:1.

9. A method according to Claim 6, carried out using a kit which comprises:—

(a) nitrocefin and

(b) competing substrates selected from each of the following classes (1) to (7)

(1) Cefoxitin, cefotaxime, moxalactam, ceftizoxime and R0-13-9904;
(2) Cefoxitin, moxalactam, R0-13-9904 and MK 0787;
(3) SQ 14,359, moxalactam and MK 0787;
(4) Cefamandole, cephradine, CP 45,899 clavulanic acid and MK 0787;
(5) Cloxacillin, cephaloridine, cefotaxime, AQ 14,359, cefamandole, mecillinam, R0-13-9904 and CP 45,899;
(6) Cefoperazone, carbenicillin, mecillinam, ceftazidime and MK 0787; and
(7) Cephaloridine, cefoxitin, SQ 14,359, CP 45,899, clavulanic acid, MK 0787 and ticarcillin.

10. A method according to Claim 9, modified in that class (3) is replaced by the following classes (3A) and (3B):—

(3A) Cefotaxime

(3B) Cefamandole, cephradine, MK 0787, CP 45,899 and clavulanic acid and in that class (5) is omitted.

11. A method according to Claim 9 or 10, modified in that class (1) is replaced by the following classes (1A) and (1B)

(1A) SQ 14,359

(1B) Moxalactam, cephradine, mecillinam, ceftizoxime, R0-13-9904, CP 45,899 and MK 0787 and in that class (7) is omitted.

12. A method according to Claim 9, 10, or 11 in which the competing substrates are further selected from any one or more of the following further classes:—

(8) cloxacillin, cefotaxime, SQ 14,359, moxalactam, carbenicillin, ceftazidime, clavulanic acid, amoxycillin and ticarcillin;
(9) ampicillin, cloxacillin, cefoxitin and cefuroxime, and
(10) ampicillin, R0-13-9904 CP45,899 and clavulanic acid.

13. A method according to Claim 12, wherein the competing substrates include at least one from class (8) and at least one of moxalactam, carbenicillin, cefamandole., mecillinam, ceftizoxime and R0-13-9904.

14. A method according to Claim 1 or 2 wherein a relative affinity is determined semi-quantitatively and the assay is monitored by visual inspection.

15. A method according to Claim 14 wherein each assay is carried out using a ratio of competing substrate to chromogenic substrate such that a visually recognisable difference in the spectrum of the chromogenic substrate occurs between competitive assays of different beta-lactamases using the same competing substrate.

16. A method according to Claim 15 wherein the relative affinity is determined as a score according to whether there is substantially no change in the spectrum of the chromogenic substrate, a small change or a large one.

17. A method according to Claim 14 carried out using a kit comprising:— nitrocefin as chromogenic substrate and all of (A) cefoxitin, (B) cefotaxime, (C) cefamandole and (D) mecillinam, as competing substrates.

18. A method according to Claim 17 wherein the kit includes a beta-lactam selected from at least one of the following groupings (F), (G) and (J):—

(F) moxalactam, cloxacillin, cefotaxime or SQ 14,359

(G) cloxacillin, cefoxitin or cefuroxime and

(J) R0-13-9904.

19. A method according to Claim 14, 15 or 16 wherein the assay is monitored spectrophotometrically and irreversible inhibition is determined.

20. A method according to Claim 19 carried out using a kit comprising:—

Nitrocefin as chromogenic substrate; and *either* all of (A/I) cefoxitin, (B) cefotaxime, (C) cefamandole, (D) mecillinam, (E/F) moxalactam, (G) cloxacillin, (H) clavulanic acid and (J) R0-13-9904 or all of (A) SQ 14,359, (B/E) moxalactam, (C) cephradine, (D) ceftazidime, (F) cloxacillin, (G/J) cefoxitin, (H) CP 45,899 and (I) clavulanic acid, as competing substrates.

21. A method according to any preceding claim wherein the beta-lactamase to be identified is extracted from bacterial obtained from a sample of human blood.

22. A kit for use in a method according to Claim 4 comprising:—

(a) at least five sets of mixtures of the chromogenic substrate and a competing substrate, each in at least 4 different ratios per set and the competing substrate being different in each set of mixtures, and

(b) the chromogenic substrate *per se*, as a control.

23. A kit according to Claim 22 wherein the chromogenic substrate is nitrocefin.

24. A kit for use in a method according to Claim 15, comprising:—

(a) at least five sets of a mixture of the chromogenic substrate with a competing substrate, wherein the competing substrate is the same or different between one set and another, but, if the same, is present in no more than two sets in different ratios, each mixture being such that a visually recognisable difference in the spectrum of the chromogenic substrate would occur between competitive assays of different beta-lactamases using the mixture, and

(b) the chromogenic substrate *per se*, as a control.

25. A kit according to Claim 24 wherein the chromogenic substrate is nitrocefin.

26. A kit according to Claim 25 containing cefoxitin in two different ratios to nitrocefin.

27. A kit according to Claim 25 containing competing substrates as defined in Claim 17 or 18.

28. A kit according to any one of Claims 22 to 27, which further includes an identified beta-lactamase for testing the kit.

**Patentansprüche**

1. Verfahren zur Identifizierung einer nicht identifizierten Beta-Lactamase, dadurch gekennzeichnet, daß man einen kompetitiven Assay an dieser Beta-Lactamase mit 1) einem chromogenes Beta-Lactam enthaltenden Substrat, das dazu befähigt ist, eine Veränderung in seinem Spektrum in sichtbarem Licht zu untergehen, wenn es unter kompetitiven Assay-Bedingungen mit Beta- Lactamasen umgesetzt wird, und 2) einem konkurrierenden Substrat für die Beta-Lactamase unterwirft, den Assay auf eine Änderung im Spektrum im sichtbaren Licht des chromogenen Substrats überwacht, daraus eine relative Affinität der konkurrierenden Substanz zu dem chromogenen Substrat auf die nicht identifizierte Beta-Lactamase bestimmt, diesen Assay wenigstens viermal wiederholt, wobei man für jeden Assay ein verschiedenes konkurrierendes Substrat oder das gleiche konkurrierende Substrat in nicht mehr als zwei verschiedenen Verhältnissen des konkurrierenden Substrats zum chromogenen Substrat verwendet und die Beta-Lactamase identifiziert, indem man auf eine Zusammenfassung der vorher bestimmten relativen Affinitäten von jedem konkurrierenden Substrat relativ zum chromogenen Substrat für verschiedenen Beta-Lactamasen Bezug nimmt.

2. Verfahren nach Anspruch 1, dadurch gekennzeichnet, daß das chromogene Substrat Nitrocefin ist.

3. Verfahren nach Anspruch 1 oder 2, dadurch gekennzeichnet, daß die relative Affinität quantitativ bestimmt wird und jeder Assay in einer Weise durchgeführt wird, welche es ermöglicht, jede irreversible Inhibierung der Beta-Lactamase durch das konkurrierende Substrat nachzuweisen.

4. Verfahren nach Anspruch 3 unter Verwendung von wenigstens zwei verschiedenen konkurrierenden Substraten, dadurch gekennzeichnet, daß jeder einzelne Assay durchgeführt wird, indem wenigstens vier verschiedene Verhältnisse von konkurrierendem Substrat zu chromogenem Substrat verwendet werden und eine relative Affinität als folgendes Verhältnis bestimmt wird:

$$\frac{\text{Konzentration von Betal-Lactam enthaltendem Substrat, das eine definierte prozentuelle Inhibierung der Spaltung des chromogenen Substrats liefert}}{\text{Konzentration an chromogenem Substrat}}$$

5. Verfahren nach Anspruch 4, dadurch gekennzeichnet, daß der definierte Prozentsatz 50% beträgt.

6. Verfahren nach Anspruch 3, 4 oder 5, dadurch gekennzeichnet, daß der Assay spektrophotometrisch überwacht wird.

7. Verfahren nach Anspruch 6, dadurch gekennzeichnet, daß die konkurrierenden Substrate zwischen den Beta-Lactamasen oder Gruppen davon um ein Verhältnis von wenigstens 2,5:1 im Index ihrer relativen Substrataffinität unterscheiden, der durch folgendes Verhältnis definiert ist:

$$\frac{\text{Konzentration von Beta-Lactam enthaltenden Substrat, das 50\% ige Inhibierung der Spaltung des chromogen Substrates liefert.}}{\text{Konzentration des chromogenen Substrats}}$$

oder sie unterscheiden je nachdem, ob sie irreversible Inhibierung liefern oder nicht.

8. Verfahren nach Anspruch 7, dadurch gekennzeichnet, daß das unterscheidende Verhältnis wenigstens 10:1 beträgt.

9. Verfahren nach Anspruch 6, durchgeführt unter Verwendung eins Kits, der enthält:
(a) Nitrocefin und
(b) konkurrierende Substrate, ausgewählt aus jeder der folgenden Klassen 1) bis 7)

(1) Cefoxitin, Cefotaxim, Moxalactam, Ceftizoxim und R0-13-9904;
(2) Cefoxitin, Moxalactam, R0-13-9904 und MK 0787;
(3) SQ 14.359, Moxalactam und MK 0787;
(4) Cefamandol, Cephradin, CP 45.899, Clavulansäure und MK 0787;
(5) Cloxacillin, Cephaloridin, Cefotaxim, AQ 14.359, Cefamandol, Mecillinam, R0-13-9904 und CP 45.899;
(6) Cefoperazon, Carbenicillin, Mecillinam, Ceftazidim und MK 0787; und
(7) Cephaloridin, Cefoxitin, SQ 14.359, CP 45.899, Clavulansäure, MK 0787 und Ticarcillin.

10. Verfahren nach Anspruch 9, dadurch gekennzeichnet, daß es modifiziert ist, indem Klasse (3) ersetzt wird durch die folgenden Klassen (3A) und (3B):
(3A) Cefotaxim
(3B) Cefamandol, Cepharadin, MK 0787, CP 45.899 und Clavulansäure, und daß Klasse (5) ausgelassen ist.

11. Verfahren nach Anspruch 9 oder 10, dadurch gekennzeichnet, daß es modifiziert ist, indem Klasse (1) ersetzt ist durch die folgenden Klassen (1A) und (1B):
(1A) SQ 14.359
(1B) Moxalactam, Cephradin, Mecillinam, Ceftizoxim, R0-13-9904, CP 45.899 und MK 0787, und daß Klasse (7) ausgelassen ist.

12. Verfahren nach Anspruch 9, 10 oder 11, dadurch gekennzeichnet, daß die konkurrierenden Substrate weiter aus einer oder mehreren der folgenden Klassen gewählt sind:

(8) Cloxacillin, Cefotaxim, SQ 14.359, Moxalactam, Carbenicillin, Ceftazidim, Clavulansäure, Amoxycillin, und Ticarcillin;
(9) Ampicillin, Cloxacillin, Cefoxitin und Cefuroxim, und
(10) Ampicillin, R0-13-9904, CP 45.899 und Clavulansäure.

13. Verfahren nach Anspruch 12, dadurch gekennzeichnet, daß die konkurrierenden Substrate wenigstens eines aus Klasse (8) und wenigstens eine der Verbindungen Moxalactam, Carbenicillin, Cefamandol, Mecillinam, Ceftizoxim und R0-13-9904 umfassen.

14. Verfahren nach Anspruch 1 oder 2, dadurch gekennzeichnet, daß eine relative Affinität semiquantitativ bestimmt und der Assay durch visuelle Inspektion überwacht wird.

15. Verfahren nach Anspruch 14, dadurch gekennzeichnet, daß jeder Assay durchgeführt wird, indem man ein Verhältnis vom konkurrierendem Substrat zu chromogenem Substrat derart verwendet, daß ein visuell erkennbarer Unterschied im Spektrum des chromogenen Substrats zwischen kompetitiven Assays von verschiedenen Beta-Lactamasen unter Verwendung des gleichen konkurrierenden Substrates auftritt.

16. Verfahren nach Anspruch 15, dadurch gekennzeichnet, daß die relative Affinität als Punktzahl bestimmt wird, gemäß welcher praktisch keine Änderung im Spektrum des chromogenen Substrates, eine geringe Änderung oder eine große Änderung vorliegt.

17. Verfahren nach Anspruch 14, dadurch gekennzeichnet, daß es unter Verwendung eines Kit durchgeführt wird, der enthält: Nitrocefin als chromagenes Substrat und alle Bestandteile (A) Cefoxitin, (B) Cefotaxim, (C) Cefamandol und (D) Mecillinam, als konkurrierende Substrate.

18. Verfahren nach Anspruch 17, dadurch gekennzeichnet, daß der Kit ein Beta-Lactam, ausgewählt aus wenigstens einer der folgenden Gruppen (F), (G) und (J), enthält:
(F) Moxalactam, Cloxacillin, Cefotaxim oder SQ 14.359
(G) Cloxacillin, Cefoxitin oder Cefuroxim, und
(J) R0-13-9904.

19. Verfahren nach Anspruch 14, 15 oder 16, dadurch gekennzeichnet, daß der Assay spektrophotometrisch überwacht und die irreversible Inhibierung bestimmt wird.

20. Verfahren nach Anspruch 19, dadurch gekennzeichnet, daß es unter Verwendung eines Kit durchgeführt wird, der enthält: Nitrocefin als chromagenes Substrat und entweder alle der Substanzen (A/I) Cefoxitin, (B) Cefotaxim, (C) Cefamandol, (D) Mecillinam, (E/F) Moxalactam, (G) Cloxacillin, (H) Clavulansäure und (J) R0-13-9904

oder alle der Substanzen (A) SQ 14.359, (B/E) Moxalactam, (C) Cephradin, (D) Ceftazidim, (F) Cloxacillin, (G/H) Cefoxitin, (H) CP 45.899 und (I) Clavulansäure als konkurrierende Substrate.

21. Verfahren nach einem der vorhergehenden Ansprüche, dadurch gekennzeichnet, daß die zu identifizierende Beta-Lactamase aus Bakterien extrahiert ist, die aus einer Probe von menschlichem Blut erhalten sind.

22. Kit zur Verwendung in einem Verfahren gemäß Anspruch 4, enthaltend:

(a) wenigstens fünf Sätze von Gemischen des chromogenen Substrates und eines konkurrierenden Substrates, jedes in wenigstens 4 verschiedenen Verhältnissen pro Satz, wobei das konkurrierende Substrat in jedem Satz von Gemischen verschieden ist, und

(b) das chromogene Substrat als solches als Kontrolle.

23. Kit nach Anspruch 22, dadurch gekennzeichnet, daß das chromogene Substrat Nitrocefin ist.

24. Kit zur Verwendung in einem Verfahren nach Anspruch 15, dadurch gekennzeichnet, daß es enthält:

(a) wenigstens fünf Sätze eines Gemisches des chromogenen Substrates mit einem konkurrierenden Substrat, wobei das konkurrierende Substrat das gleiche ist oder zwischen einem Satz und einem anderen verschieden ist, jedoch, wenn as das gleiche ist, in nicht mehr als zwei Sätzen in verschiedenen Verhältnissen vorliegt, wobei jedes Gemisch so ist, daß ein visuell erkennbarer Unterschied im Spektrum des chromogenen Substrates zwischen konkurrierenden Assays von verschiedenen Beta-Lactamasen unter Verwendung des Gemisches aufträte, und

(b) das chromogene Substrat als solches als Kontrolle.

25. Kit nach Anspruch 24, dadurch gekennzeichnet, daß das chromogene Substrat Nitrocefin ist.

26. Kit nach Anspruch 25, dadurch gekennzeichnet, daß er Cefoxitin in zwei verschiedenen Verhältnissen zu Nitrocefin enthält.

27. Kit nach Anspruch 25, dadurch gekennzeichnet, daß er konkurrierende Substrate nach Anspruch 17 oder 18 enthält.

28. Kit nach einem der Ansprüche 22 bis 27, dadurch gekennzeichnet, daß er weiter eine identifizierte Beta-Lactamase zur Prüfung des Kit enthält.

**Revendications**

1. Procédé pour identifier une bêta-lactamase non identifiée, ce procédé comprenant la réalisation d'un essai de détermination compétitive sur ladite bêta-lactamase avec (1) un substrat contenant du bêta-lactame chromogène, capable de subir une modification de son spectre en lumière visible quand il a été mis en réaction, dans les conditions d'un essai de détermination compétitive, avec des bêta-lactamases, et (2) un substrat pouvant entrer en compétition pour la bêta-lactamase, pa surveillance de l'essai de détermination pour déceler une variation, dans le spectre de la lumière visible, du substrat chromogène, la détermination, à partir de cette variation, d'une affinité relative du substrat compétiteur, par rapport au substrat chromogène, pour la bêta-lactamase non identifiée, la répétition dudit essai de détermination au moins quatre fois en utilisant pour chaque essai de détermination un substrat compétiteur différent ou bien le même substrat compétiteur en pas plus de deux rapports différents du substrat compétiteur au substrat chromogène, et l'identification de la bêta-lactamase par référence à une compilation d'affinités relatives, préalablement déterminées de chaque substrat compétiteur par rapport au substrat chromogène pour différentes bêta-lactamases.

2. Procédé selon la revendication 1, dans lequel le substrat chromogène est la nitrocéfine.

3. Procédé selon la revendication 1 ou 2, fans lequel l'affinité relative est déterminée quantitativement, et l'on effectue chaque essai de détermination d'une manière permettant de déceler toute inhibition irréversible éventuelle de la bête-lactamase par le substrat compétiteur.

4. Procédé selon la revendication 3, en utilisant au moins deux substrats compétiteurs différents, dans lequel on effectue chaque essai individuel de détermination en utilisant au moins quatre rapports différents du substrat compétiteur au substrat chromogène, et l'on détermine une affinité relative comme étant le rapport:

$$\frac{\text{Concentration du substrat contenant un groupe bêta-lactame qui donne un pourcentage défini d'inhibition du clivage du substrat chromogène}}{\text{Concentration du substrat chromogène}}$$

5. Procédé selon la revendication 4, dans lequel le pourcentage défini est égal à 50%.

6. Procédé selon la revendication 3, 4 ou 5, dans lequel l'essai de détermination est surveillé par spectrophotométrie.

7. Procédé selon la revendication 6, dans lequel les substrats compétiteurs vont distinguer entre les bêta-lactamases ou leurs groupes présentant un rapport d'au moins 2,5:1 de lauer indice d'affinité relative de substrat, défini par le rapport:

$$\frac{\text{Concentration du substrat contenant du bêta-lactame qui donne 50\% d'inhibition de clivage du substrate chromogène}}{\text{Concentration du substrate chromogène}}$$

ou bien ils vont les distinguer selon qu'ils ou elles donnent ou non une inhibition irréversible.

8. Procédé selon la revendication 7, dans lequel le rapport permettant la distinction est au moins égal à 10:1.

9. Procédé selon la revendication 6, effectué en utilisant une trousse qui comprend:
(a) la nitrocéfine, et
(b) des substrats compétiteurs choisis parmi chacune des classes (1) à (7) suivantes:

(1) la céfoxitine, le céfotaxime, le moxalactame, le ceftizoxime et R0-13 9904;
(2) la céfoxitine, le moxalactame, R0-13-9904 et MK 0787;
(3) SQ 14359, le moxalactame et MK 0787;
(4) le céfamandole, la céphradine, CP 45 899, l'acide clavulanique et MK 0787;
(5) la cloxacilline, la céphaloridine, le céfotaxime, AQ 14 359, le céfamandole, le mécilliname, R0-13-9904 et CP 45 899;
(6) la céfopérazone, la carbénicilline, le mécilliname, la ceftazidime et MK 0787; et
(7) la céphaloridine, la céfoxitine, SQ 14 359, CP 45 899, l'acide clavulanique, MK 0787 et la tricarcilline.

10. Procédé selon la revendication 9, modifié en ce que la classe (3) est remplacée par les classes (3A) et (3B) suivantes:
(3A) le céfotaxime
(3B) le céfamandole, la céphradine, MK 0787, CP 45 899 et l'acide clavunalique, et en ce que la classe (5) est omise.

11. Procédé selon la revendication 9 ou 10, modifié en ce que la classe (1) est remplacée par les classes (1A) et (1B) suivantes:
(1A) SQ 14 359
(1B) le oxalactame, la céphradine, le mécilliname, la ceftizoxime, R0-13-9904, CP 45 899 et MK 0787, et en ce que la classe (7) est omise.

12. Procédé selon la revendication 9, 10 ou 11, dans lequel les substrats compétiteurs sont en outre choisis parmi l'une quelconque ou plus d'une des autres classes suivantes:

(8) la cloxacilline, le céfotaxime, SQ 14 359, le moxalactame, la carbénicilline, le ceftazidime, l'acide clavulanique, l'amoxycilline et la ticarcilline;
(9) l'ampicilline, la cloxacilline, la céfoxitine et le céfuroxime, et
(10) l'ampicilline, R0-13-9904, CP 45 899 et l'acide clavulanique.

13. Procédé selon la revendication 12, dans lequel les substrats compétiteurs comprennent au moins un substrat provenant de la classe (8) et au moins un substrat qui est le moxalactame, la carbénicilline, le céfamandole, le mécilliname, le ceftizoxime et R0-13-9904.

14. Procédé selon la revendication 1 ou 2, dans lequel on détermine semi-quantativement une affinité relative et l'on surveille par examen visuel l'essai de détermination.

15. Procédé selon la revendication 14, dans lequel on effectue chaque essai de détermination en utilisant un rapport du substrat compétiteur au substrat chromogène tel qu-il se produise une différence, reconnaissable visuellement, dans le spectre du substrat chromogène entre les essais compétitifs de détermination des différentes bêta-lactames quand on utilise le même substrat compétiteur.

16. Procédé selon la revendication 15, dans lequel on détermine l'affinité relative sous forme d'une cotation attribuée selon qu'il n'y a essentiellement pas de modification dans le spectre du substrat chromogène, qu'il existe une faible modification ou une grande modification.

17. Procédé selon la revendication 14, mis en oeuvre en utilisant une trousse comprenant:
la nitrocéfine comme substrat chromogène et la totalité de:
(A) la céfoxitine, (B) le céfotaxime, (C) le céfamandole et (D) le mecilliname, à titre de substrats compétiteurs.

18. Procédé selon la revendication 17, dans lequel la trousse comprend un bêta-lactame choisi parmi au moins l'un des groupements (F), (G) et (J) qui suivent:
(F) le moxalactame, la cloxacilline, le céfotaxime ou SQ 14 359,

(G) la cloxacilline, la céfoxitine ou le céfuroxime et

(J) R0-13-9904.

19. Procédé selon la revendication 14, 15 ou 16, dans lequel l'essai de détermination est surveillé par spectrophotométrie et on détermine une inhibition irréversible.

20. Procédé selon la revendication 19, effectué à l'aide d'une trousse comprenant:

la nitrocéfine comme substrat chromogène; et

soit la totalité des substrats (A/I) de la céfoxitine, (B) la céfotaxime, (C) le céfamandole, (D) le mécilliname, (E/F) le moxalactame, (G) la cloxacilline, (H) l'acide clavulanique, et (J) R0-13-9904,

soit la totalité des substrats (A) SQ-14 359, (B/E) le moxalactame, (C) la céphradine, (D) le ceftazidime, (F) la cloxacilline, (G/J) la céfoxitine, (H) CP 45 899 et (I) l'acide clavulanique, à titre de substrats compétiteurs.

21. Procédé selon l'une quelconque des revendications précédentes, dans lequel la bêta-lactamase à identifier est extraite de bactéries obtenues sur un échantillon de sang humain.

22. Trousse à utiliser dans un procédé selon la revendication 4, comprenant:

(a) au moins cinq groupes de mélanges du substrat chromogène et d'un substrat compétiteur, chacun en au moins quatre rapports différents par groupe, et le substrat compétiteur étant différent dans chaque groupe de mélanges, et

(b) le substrat chromogène lui-même, à titre de témoin.

23. Trousse selon la revendication 22, dans laquelle le substrat chromogène est la nitrocéfine.

24. Trousse destinée à être utilisée dans un procédé selon la revendication 15, comprenant:

(a) au moins cinq groupes d'un mélange du substrat chromogène avec un substrat compétiteur, le substrat compétiteur étant le même d'un groupe à un autre ou étant différent d'un groupe à un autre mais, s'il est le même, ce substrat n'étant présent dans pas plus de deux groupes en des rapports différents, chaque mélange étant tel qu'une différence, reconnaissable visuellement, dans le spectre du substrat chromogène va se produire entre des essais de détermination avec compétition de différentes bêta-lactamases quand on utilise le mélange, et

(b) le substrat chromogène lui-même, à titre de témoin.

25. Trousse selon la revendication 24, dans laquelle le substrat chromogène est la nitrocéfine.

26. Trousse selon la revendication 25, contenant la céfoxitine en deux rapports différents par rapport à la nitrocéfine.

27. Trousse selon la revendication 25, contenant les substrats compétiteurs tels que définis dans la revendication 17 ou 18.

28. Trousse selon l'une quelconque des revendications 22 à 27, qui comprend en outre une bêta-lactamase identifiée, pour tester la trousse.

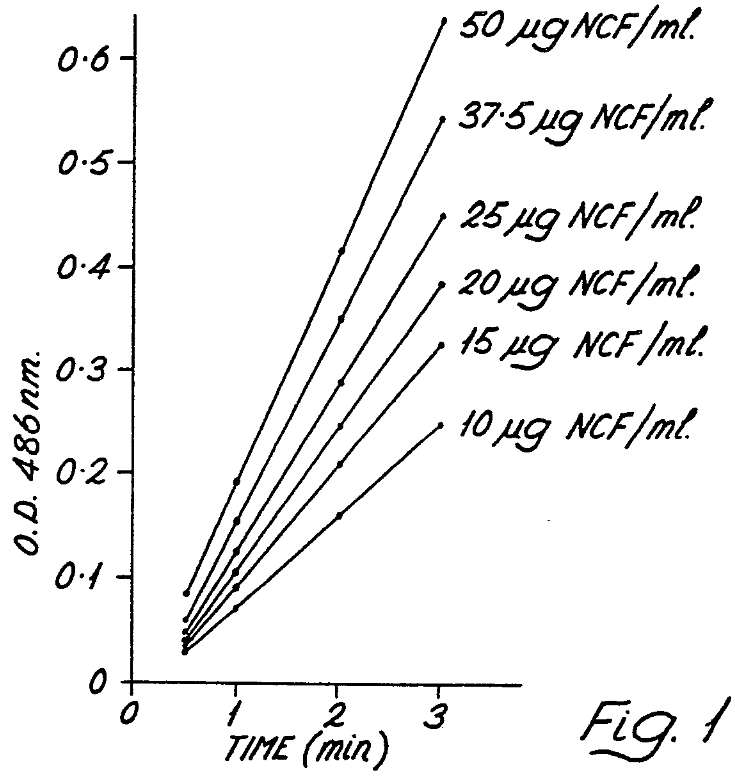
50 μg NCF/ml.
37·5 μg NCF/ml.
25 μg NCF/ml.
20 μg NCF/ml.
15 μg NCF/ml.
10 μg NCF/ml.
O.D. 486nm.
0·6
0·5
0·4
0·3
0·2
0·1
0
0
1
2
3
TIME (min)

Fig. 1

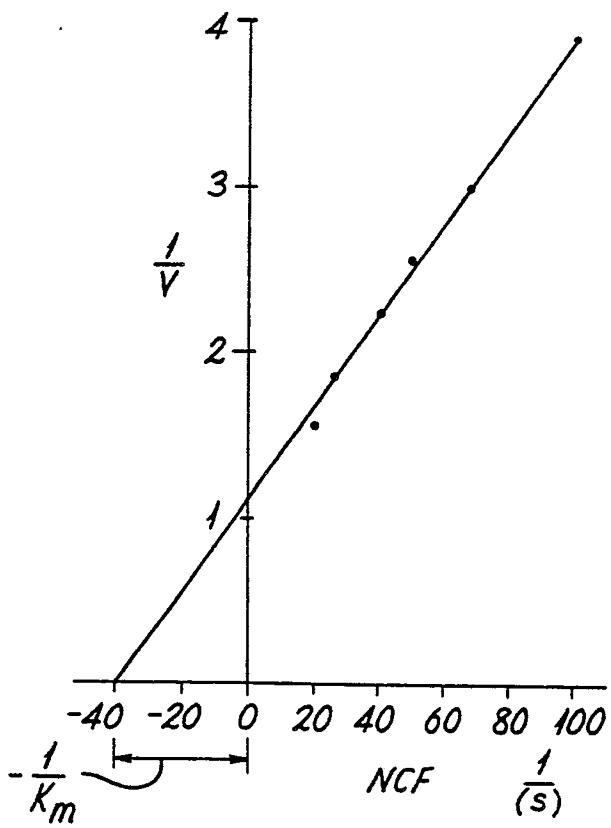
4
3
2
1
1/V
-40
-20
0
20
40
60
80
100
NCF
1/(s)
-1/Km

Fig. 2

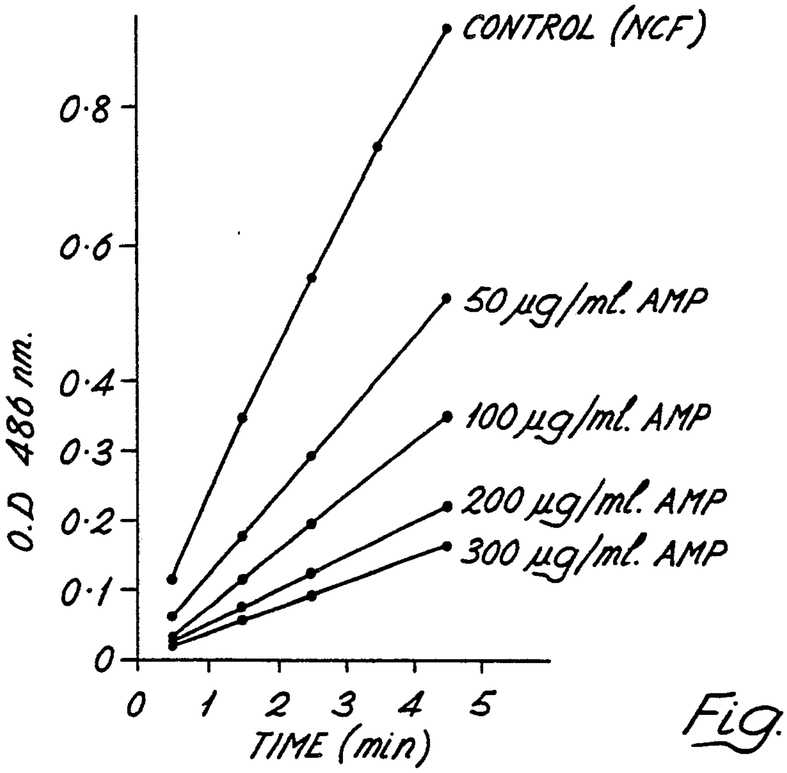
CONTROL (NCF)
50 µg/ml. AMP
100 µg/ml. AMP
200 µg/ml. AMP
300 µg/ml. AMP
O.D 486 nm.
0·8
0·6
0·4
0·3
0·2
0·1
0
0 1 2 3 4 5
TIME (min)

Fig. 3

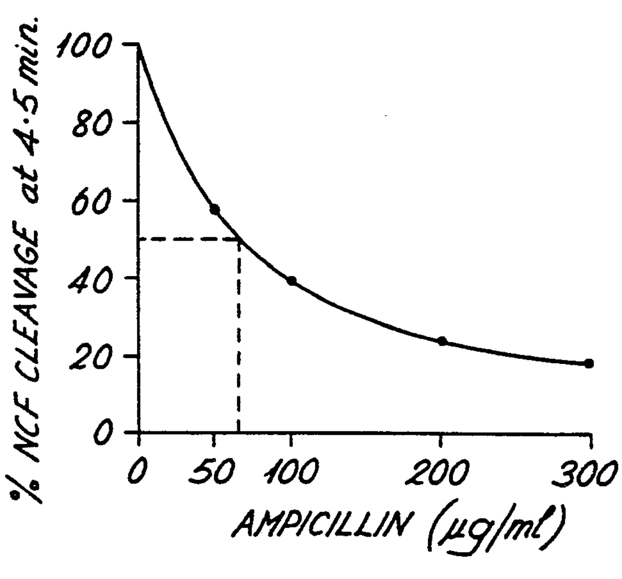
% NCF CLEAVAGE at 4·5 min.
100
80
60
40
20
0
0 50 100 200 300
AMPICILLIN (µg/ml)

Fig. 4

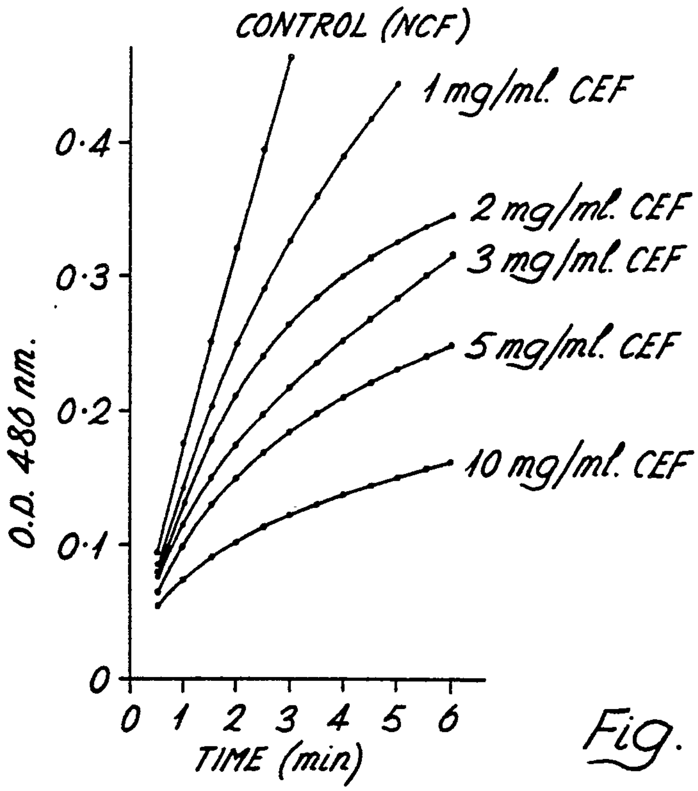
CONTROL (NCF)
1 mg/ml. CEF
2 mg/ml. CEF
3 mg/ml. CEF
5 mg/ml. CEF
10 mg/ml. CEF
O.D. 486 nm.
0·4
0·3
0·2
0·1
0
0 1 2 3 4 5 6
TIME (min)

Fig. 5

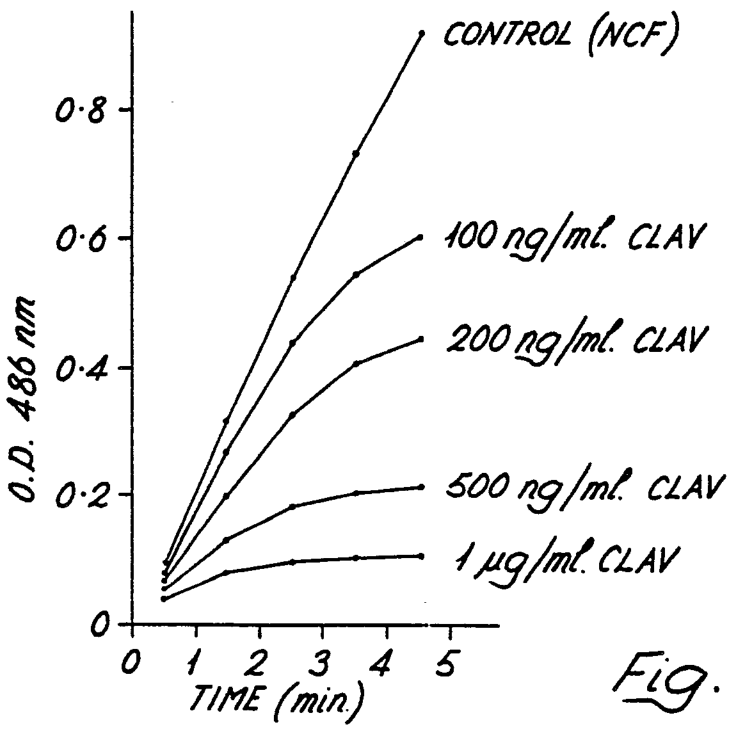
CONTROL (NCF)
100 ng/ml. CLAV
200 ng/ml. CLAV
500 ng/ml. CLAV
1 μg/ml. CLAV
O.D. 486 nm
0·8
0·6
0·4
0·2
0
0 1 2 3 4 5
TIME (min.)

Fig. 6

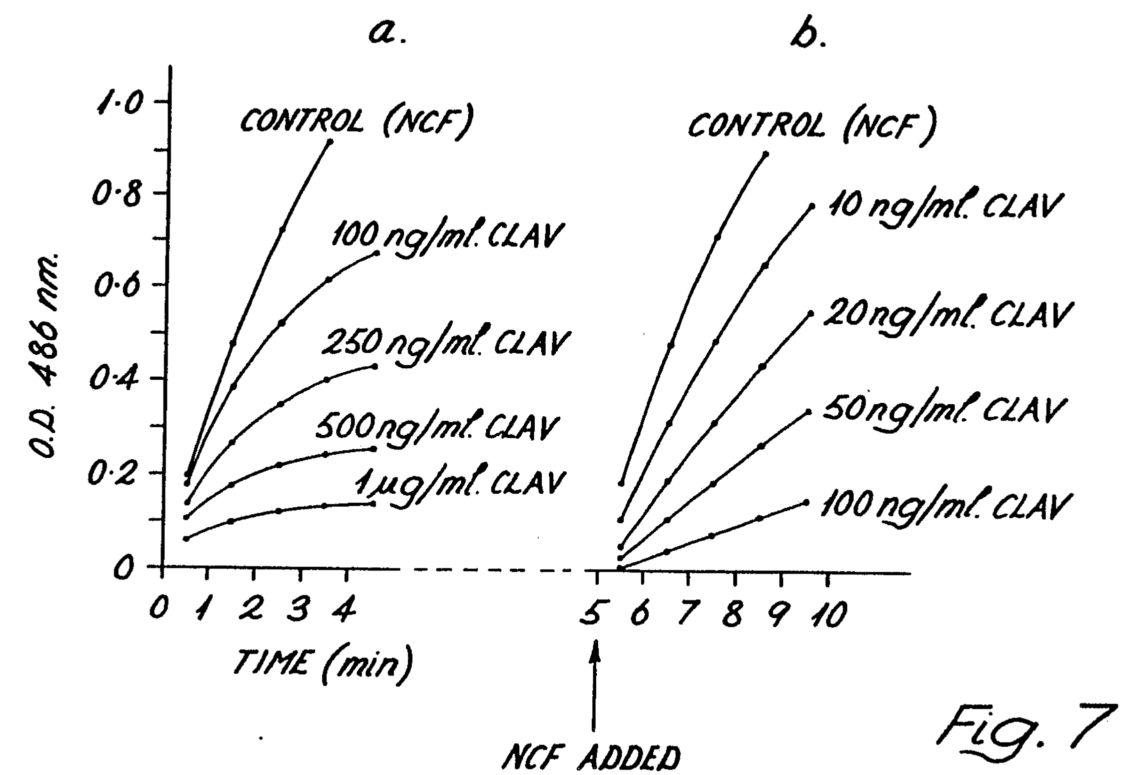
a.
b.
O.D. 486 nm.
1·0
0·8
0·6
0·4
0·2
0
CONTROL (NCF)
100 ng/ml. CLAV
250 ng/ml. CLAV
500 ng/ml. CLAV
1 μg/ml. CLAV
CONTROL (NCF)
10 ng/ml. CLAV
20 ng/ml. CLAV
50 ng/ml. CLAV
100 ng/ml. CLAV
0 1 2 3 4
5 6 7 8 9 10
TIME (min)
NCF ADDED

Fig. 7

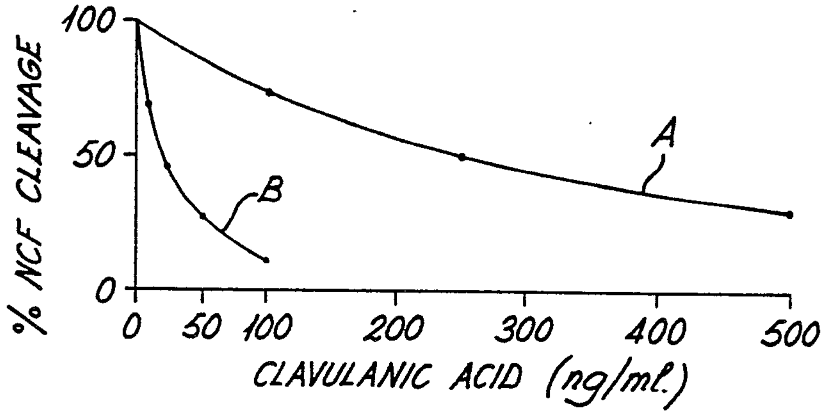
100
50
0
% NCF CLEAVAGE
A
B
0
50
100
200
300
400
500
CLAVULANIC ACID (ng/ml)

Fig. 8

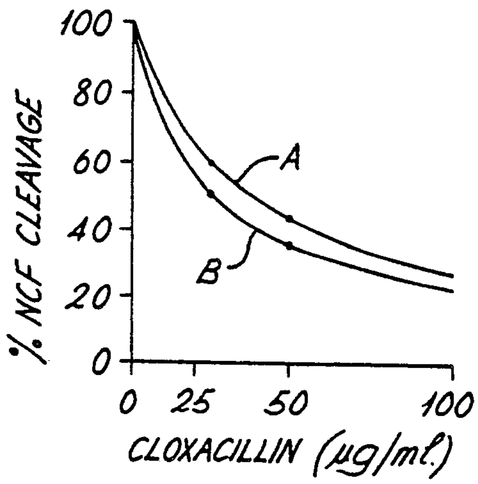
100
80
60
40
20
0
% NCF CLEAVAGE
A
B
0
25
50
100
CLOXACILLIN (µg/ml)

Fig. 9